# EUROPEAN PATENT APPLICATION

(11) **EP 4 011 378 A1**
(43) Date of publication of application: **15.06.2022**
(21) Application number: 20849500.2
(22) Date of filing: 05.08.2020
(51) Int. Cl.: A61K 31/702, A61P 3/10, A61P 19/02

(54) **USE OF MANNURONIC ACID OLIGOSACCHARIDES OR COMPOSITION COMPRISING SAME IN TREATMENT OF TH1-DOMINANCE RELATED DISEASES**

(30) Priority: 06.08.2019 CN 201910719369
(71) Applicant: Shanghai Green Valley Pharmaceutical Co., Ltd., Pudong New Area, Shanghai 201203 (CN); Shanghai Institute Materia Medica, Chinese Academy Of Sciences, Pudong, Shanghai 201203 (CN)
(72) Inventor: GENG, Meiyu, Shanghai 201203 (CN); SUN, Guangqiang, Shanghai 201203 (CN); WANG, Xinyi, Shanghai 201203 (CN); ZHANG, Jing, Shanghai 201203 (CN); FENG, Teng, Shanghai 201203 (CN)
(74) Representative: Murgitroyd & Company
(86) International application number: PCT/CN2020/107225
(87) International publication number: WO 2021/023239

(57) **Abstract**

A use that relates to mannuronic acid oligosaccharides or a composition comprising same in the treatment of Th1-dominance related diseases. The Th1-dominance related diseases may be selected from Multiple Sclerosis, Crohn's disease, Type I Diabetes, Rheumatoid Arthritis, Hashimoto's thyroiditis, vitiligo, Sjögren's Syndrome, Polycystic ovary syndrome, Celiac Disease and Grave's disease, preferably from Multiple Sclerosis, Crohn's disease and Rheumatoid Arthritis.

## Description

### TECHNICAL FIELD

The present invention relates to the use of mannuronic acid oligosaccharides or compositions comprising the same to treat Th1-dominance related diseases.

### BACKGROUND OF THE INVENTION

Naive T cells can differentiate into T helper cell type 1 (Th1 cells) and T helper cell type 2 (Th2 cells) in vivo. The abnormal imbalance of Th1 cells relative to Th2 cells in vivo is related to some diseases. Such an imbalance includes a state dominated by Th1 cells, i.e., the naive T cells are overdifferentiated into Th1 cells, and a state dominated by Th2 cells, i.e., the naive T cells are overdifferentiated into Th2 cells. Th1-dominance related diseases include, but are not limited to, Multiple Sclerosis, Crohn's Disease, Type 1 Diabetes, Rheumatoid Arthritis, Hashimoto's thyroiditis, Vitiligo, Sjögren's Syndrome, Polycystic ovarian syndrome (PCOS), Celiac Disease, Graves's disease.

Mannouronic acid oligosaccharides have received extensive attention due to their potential medicinal value. Mannouronic acid oligosaccharides are usually prepared through multiple steps using alginic acid as a raw material.

The polysaccharide molecule of the raw material, alginic acid, comprises an M segment formed of D-mannuronic acids linked by β-1,4-glucosidic bonds, a G segment formed of L-guluronic acids linked by α-1,4-glucosidic bonds, and an MG segment formed by hybridization of the two sacchorides. The structural formula of D-mannuronic acid and L-guluronic acid are shown in the following Formula (I):

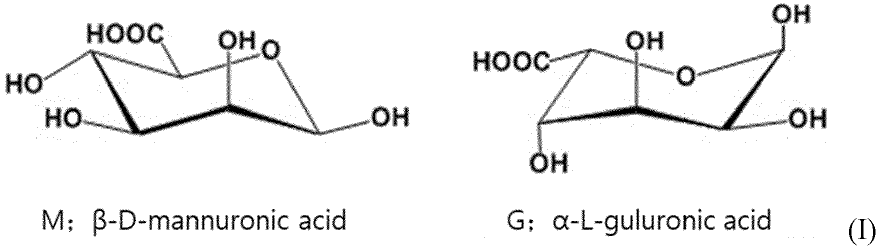

The M segment and the G segment can be separated from the raw material, alginic acids. A common method can be simply described below: alginic acid is preliminarily degraded to give mixed polysaccharides of polymannuronic acid and polyguluronic acid; then the mixed polysaccharides are subjected to acidic precipitation to remove the polyguluronic acid therein, and further refinement is conducted to obtain a homopolymannuronic acid with a purity of more than 90% (hereinafter also referred to as "M-segment intermediate"). For the method, see, for example, Chinese patent application CN98806637.8 ("Method for preparing uronic acid block from alginate") and CN02823707.2 ("Method for preparing alginate with high mannuronic acid content"), which are incorporated herein by reference in their entirety.

A common preparation method of oligomeric mannuronic acid is as follows: the M-segment intermediate obtained above is subjected to further acidolysis by heating under an acidic condition to obtain a small fragment mannuronic acid polymer having a desired molecular weight range. In addition, the degradation efficiency can be improved by an oxidative degradation method; meanwhile, the reducing end can be oxidized to a ring-opened saccharic diacid, see Chinese Patent Application No. CN200580009396.5 (Patent literature 1) filed by Meiyu Geng, et al. and US Patent No. 8,835,403 B2 (Patent literature 2) for details, which are incorporated herein by reference in their entirety. For convenience of description, Patent literatures 1 and 2 are hereinafter collectively referred to as prior documents.

The reaction process of mannuronic acid oligosaccharides disclosed in the prior patent can be expressed by the following reaction equation (II): i.e., the aldehyde group at the C1-position of mannuronic acid at the reducing end of the oligomannuronic acid polysaccharide is oxidized to a carboxyl group.

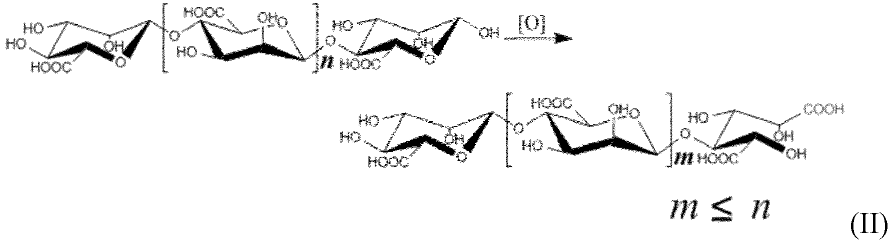

In the above-mentioned oxidation conversion process, the commonly used oxidant is alkaline copper sulfate solution, i.e., film reagent. The prior patent adopted this oxidation method, specifically: under alkaline conditions, the reaction substrate polymannuronic acid, which is the intermediate M fragemtn above, is added to the copper sulfate solution, and reacted in a boiling water bath for 15 minutes to 2 hours. This method uses Cu2+ ions as the oxidant to oxidize the aldehyde group, and a brick red cuprous oxide precipitate is produced during the reaction. This reaction is often used to identify reducing saccharides.

The prior patent CN2016100697039 discloses a preparation method of oligomannuronic acid oligosaccharides, the prior patent CN2017107964853 discloses a method for determining the weight average molecular weight and content of mannuronic acids, the prior patent CN2017114675966 discloses the composition of mannuronic acid oligosaccharides and the preparation thereof, all of which are incorporated herein by reference in their entirety.

There is a need in the art to provide new products and methods for the treatment of Th1-dominance related diseases.

### SUMMARY OF THE INVENTION

The present invention satisfies the above-mentioned needs by providing strategies for using mannuronic acid oligosaccharides or compositions comprising the same to treat Th1-dominance related diseases.

In one aspect, the present invention provides the use of a mannuronic acid oligosaccharide in the manufacture of a medicament for the treatment of Th1-dominance related diseases, wherein the mannuronic acid oligosaccharide is a mannuronic acid oligosaccharide having formula (I) or a pharmaceutically acceptable salt thereof: 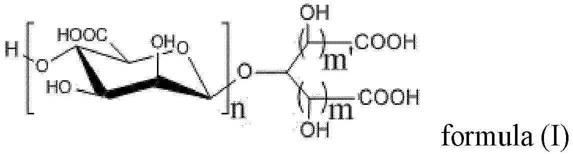 wherein n is an integer selected from 1-20, m is selected from 0, 1 or 2, and m' is selected from 0 or 1.

In another aspect, the present invention provides the use of a composition comprising mannuronic acid oligosaccharides in the manufacture of a medicament for the treatment of Th1-dominance related diseases, wherein the composition comprises a mannuronic acid oligosaccharide having formula (I) or a pharmaceutically acceptable salt thereof: 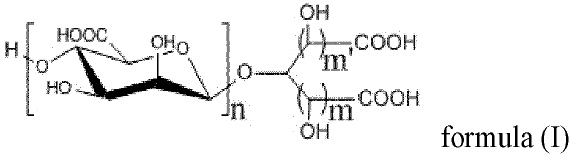
wherein n is an integer selected from 1-20, m is selected from 0, 1 or 2, m'is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

In still another aspect, the present invention provides a method of treating a patient having Th1-dominance related disease, comprising administering to the patient a therapeutically effective amount of the composition comprising mannuronic acid oligosaccharide of the present invention or the mannuronic acid oligosaccharide of the present invention.

In still another aspect, the present invention provides a composition comprising mannuronic acid oligosaccharide of the present invention or the mannuronic acid oligosaccharide of the present invention, for use in the treatment of Th1-dominance related diseases.

The applicant found that the mannuronic acid oligosaccharide or the composition comprising the mannuronic acid oligosaccharide of the present invention has a beneficial effect in the treatment of Th1-dominance related diseases, and also reduces the cost of production. Also, due to its safety derived from natural products, it is beneficial to improve the quality of life of patients.

### BRIEF DESCRIPTION OF THE DRAWINGS

Figure 1 is a mass spectrum of disaccharides, trisaccharides and tetrasaccharides in product A.
Figure 2 is a mass spectrum of pentasaccharides, hexasaccharides and heptasaccharides in product A.
Figure 3 is the mass spectrum of the octasaccharides, nonasaccharides and decasaccharides in product A.

### DETAILED DESCRIPTION OF THE INVENTION

Various aspects of the present invention will be described in detail below, but the present invention is not limited to these specific embodiments. Those skilled in the art can make some modifications and adjustments to the present invention according to the the substantial disclosure below, and these adjustments are also within the scope of the present invention.

The present invention provides the following embodiments.

### Embodiment A regarding Th1-dominance related diseases

A1. The use of a composition comprising mannuronic acid oligosaccharides in the manufacture of a medicament for the treatment of Th1-dominance related diseases, wherein the composition comprises a mannuronic acid oligosaccharide having formula (I) or a pharmaceutically acceptable salt thereof: 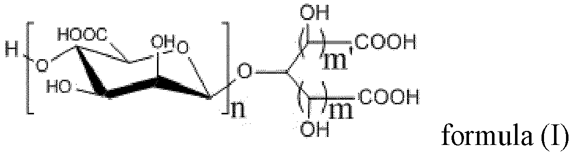
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

A2. The use according to embodiment A1, wherein the total weight of mannuronic acid oligosaccharides with n=1-2 accounts for 10-50%, preferably 25-50% of total weight of the composition.

A3. The use according to embodiment A1, wherein the ratio of the total weight of mannuronic acid oligosaccharides with n=1-3 to the total weight of mannuronic acid oligosaccharides with n=4-7 is between 1.0-3.5.

A4. The use according to embodiment A1, wherein the total weight of mannuronic acid oligosaccharides with m+m'=1 or 2 is not less than 50%, preferably 60%-90%, more preferably 70%-90% of total weight of the composition.

A5. The use according to embodiment A4, wherein the total weight of mannuronic acid oligosaccharides with m+m'=1 is not less than 10%, preferably 30-40% of total weight of the composition.

A6. The use according to embodiment A4, wherein the total weight of mannuronic acid oligosaccharides with m+m'=2 is not less than 10%, preferably 30-50% of total weight of the composition.

A7. The use according to embodiment A1, wherein the total weight of mannuronic acid oligosaccharides with n=1-5 accounts for 80-95% of total weight of the composition.

A8. The use according to embodiment A1, wherein the total weight of mannuronic acid oligosaccharides with n=1-3 accounts for 20-70% of total weight of the composition.

A9. The use according to embodiment A3, wherein the ratio of the total weight of mannuronic acid oligosaccharides with n=1-3 to the total weight of mannuronic acid oligosaccharides with n=4-7 is between 1.0-3.0.

A10. The use according to any one of embodiments A1-A9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

A11. The use according to embodiment A10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

A12. The use according to embodiment A11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

A13. The use according to embodiment A1, wherein n is an integer selected from 1-19.

A14. The use according to embodiment A1, wherein n is an integer selected from 1-18.

A15. The use according to embodiment A1, wherein n is an integer selected from 1-17.

A16. The use according to embodiment A1, wherein n is an integer selected from 1-16.

A17. The use according to embodiment A1, wherein n is an integer selected from 1-15.

A18. The use according to embodiment A1, wherein n is an integer selected from 1-14.

A19. The use according to embodiment A1, wherein n is an integer selected from 1-13.

A20. The use according to embodiment A1, wherein n is an integer selected from 1-12.

A21. The use according to embodiment A1, wherein n is an integer selected from 1-11.

A22. The use according to embodiment A1, wherein n is an integer selected from 1-10.

A23. The use according to embodiment A1, wherein n is an integer selected from 1-9.

A24. The use of a mannuronic acid oligosaccharide in the manufacture of a medicament for the treatment of Th1-dominance related diseases, wherein the mannuronic acid oligosaccharide is a mannuronic acid oligosaccharide having formula (I) or a pharmaceutically acceptable salt thereof: 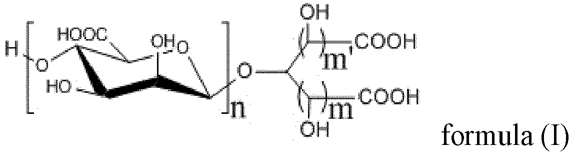 wherein n is an integer selected from 1-20, m is selected from 0, 1 or 2, and m' is selected from 0 or 1.

A25. The use according to embodiment A24, wherein n is an integer selected from 1-19.

A26. The use according to embodiment A24, wherein n is an integer selected from 1-18.

A27. The use according to embodiment A24, wherein n is an integer selected from 1-17.

A28. The use according to embodiment A24, wherein n is an integer selected from 1-16.

A29. The use according to embodiment A24, wherein n is an integer selected from 1-15.

A30. The use according to embodiment A24, wherein n is an integer selected from 1-14.

A31. The use according to embodiment A24, wherein n is an integer selected from 1-13.

A32. The use according to embodiment A24, wherein n is an integer selected from 1-12.

A33. The use according to embodiment A24, wherein n is an integer selected from 1-11.

A34. The use according to embodiment A24, wherein n is an integer selected from 1-10.

A35. The use according to embodiment A24, wherein n is an integer selected from 1-9.

A36. The use according to any one of embodiments A1-A35, wherein the pharmaceutically acceptable salt is a sodium salt or a potassium salt.

A37. A method for treating a patient having Th1-dominance related disease, comprising administering to the patient a therapeutically effective amount of the composition comprising mannuronic acid oligosaccharides defined according to any one of embodiments A1-A23 and A36 or the mannuronic acid oligosaccharide defined according to any one of embodiments A24-A36.

A38. The composition comprising mannuronic acid oligosaccharides defined according to any one of embodiments A1-A23 and A36 or the mannuronic acid oligosaccharides defined according to any one of embodiments A24-A36, for use in the treatment of Th1-dominance related diseases.

A39. The use according to any one of embodiments A1-A36 or the method according to embodiment A37 or A38, wherein the Th1-dominance related disease is selected from Multiple Sclerosis, Crohn's Disease, Type 1 Diabetes, Rheumatoid Arthritis, Hashimoto's thyroiditis, Vitiligo, Sjögren's Syndrome, polycystic ovary syndrome, Celiac Disease, Grave's disease, preferably Multiple Sclerosis, Crohn's Disease and Rheumatoid Arthritis.

### Embodiment B regarding Multiple Sclerosis

B1. The use of a composition comprising mannuronic acid oligosaccharides in the manufacture of a medicament for the treatment of Multiple Sclerosis, wherein the composition comprises a mannuronic acid oligosaccharide having formula (I) or a pharmaceutically acceptable salt thereof: 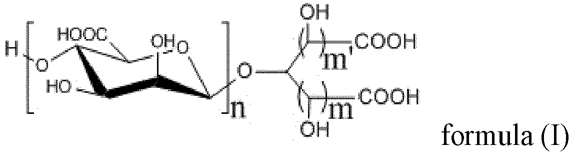
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

B2. The use according to embodiment B1, wherein the total weight of mannuronic acid oligosaccharides with n=1-2 accounts for 10-50%, preferably 25-50% of total weight of the composition.

B3. The use according to embodiment B1, wherein the ratio of the total weight of mannuronic acid oligosaccharides with n=1-3 to the total weight of mannuronic acid oligosaccharides with n=4-7 is between 1.0-3.5.

B4. The use according to embodiment B1, wherein the total weight of mannuronic acid oligosaccharides with m+m'=1 or 2 is not less than 50%, preferably 60%-90%, more preferably 70%-90% of total weight of the composition.

B5. The use according to embodiment B4, wherein the total weight of mannuronic acid oligosaccharides with m+m'=1 is not less than 10%, preferably 30-40% of total weight of the composition.

B6. The use according to embodiment B4, wherein the total weight of mannuronic acid oligosaccharides with m+m'=2 is not less than 10%, preferably 30-50% of total weight of the composition.

B7. The use according to embodiment B1, wherein the total weight of mannuronic acid oligosaccharides with n=1-5 accounts for 80-95% of total weight of the composition.

B8. The use according to embodiment B1, wherein the total weight of mannuronic acid oligosaccharides with n=1-3 accounts for 20-70% of total weight of the composition.

B9. The use according to embodiment B3, wherein the ratio of the total weight of mannuronic acid oligosaccharides with n=1-3 to the total weight of mannuronic acid oligosaccharides with n=4-7 is between 1.0-3.0.

B10. The use according to any one of embodiments B1-B9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

B11. The use according to embodiment B10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

B12. The use according to embodiment B11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

B13. The use according to embodiment B1, wherein n is an integer selected from 1-19.

B14. The use according to embodiment B1, wherein n is an integer selected from 1-18.

B15. The use according to embodiment B1, wherein n is an integer selected from 1-17.

B16. The use according to embodiment B1, wherein n is an integer selected from 1-16.

B17. The use according to embodiment B1, wherein n is an integer selected from 1-15.

B18. The use according to embodiment B1, wherein n is an integer selected from 1-14.

B19. The use according to embodiment B1, wherein n is an integer selected from 1-13.

B20. The use according to embodiment B1, wherein n is an integer selected from 1-12.

B21. The use according to embodiment B1, wherein n is an integer selected from 1-11.

B22. The use according to embodiment B1, wherein n is an integer selected from 1-10.

B23. The use according to embodiment B1, wherein n is an integer selected from 1-9.

B24. The use of mannuronic acid oligosaccharide in the manufacture of a medicament for the treatment of Multiple Sclerosis, wherein the mannuronic acid oligosaccharide is a mannuronic acid oligosaccharide having formula (I) or a pharmaceutically acceptable salt thereof: 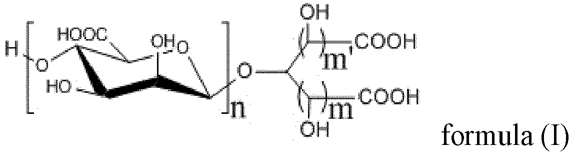 wherein n is an integer selected from 1-20, m is selected from 0, 1 or 2, and m' is selected from 0 or 1.

B25. The use according to embodiment B24, wherein n is an integer selected from 1-19.

B26. The use according to embodiment B24, wherein n is an integer selected from 1-18.

B27. The use according to embodiment B24, wherein n is an integer selected from 1-17.

B28. The use according to embodiment B24, wherein n is an integer selected from 1-16.

B29. The use according to embodiment B24, wherein n is an integer selected from 1-15.

B30. The use according to embodiment B24, wherein n is an integer selected from 1-14.

B31. The use according to embodiment B24, wherein n is an integer selected from 1-13.

B32. The use according to embodiment B24, wherein n is an integer selected from 1-12.

B33. The use according to embodiment B24, wherein n is an integer selected from 1-11.

B34. The use according to embodiment B24, wherein n is an integer selected from 1-10.

B35. The use according to embodiment B24, wherein n is an integer selected from 1-9.

B36. The use according to any one of embodiments B1-B35, wherein the pharmaceutically acceptable salt is a sodium salt or a potassium salt.

B37. A method for treating a patient having Multiple Sclerosis, the method comprises administering to the patient a therapeutically effective amount of the composition comprising mannuronic acid oligosaccharides defined according to any one of embodiments B1-B23 and B36 or the mannuronic acid oligosaccharide defined according to any one of embodiments B24-B36.

B38. The composition comprising mannuronic acid oligosaccharides defined according to any one of embodiments B1-B23 and B36 or the mannuronic acid oligosaccharides defined according to any one of embodiments B24-B36, for use in the treatment of Multiple Sclerosis.

### Embodiment C regarding Crohn's Disease

C1. The use of a composition comprising mannuronic acid oligosaccharides in the manufacture of a medicament for the treatment of Crohn's Disease, wherein the composition comprises a mannuronic acid oligosaccharide having formula (I) or a pharmaceutically acceptable salt thereof: 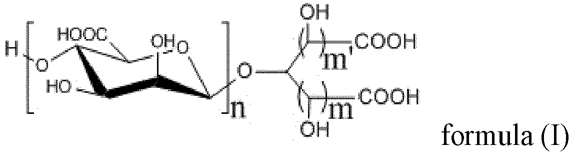
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

C2. The use according to embodiment C 1, wherein the total weight of mannuronic acid oligosaccharides with n=1-2 accounts for 10-50%, preferably 25-50% of total weight of the composition.

C3. The use according to embodiment C1, wherein the ratio of the total weight of mannuronic acid oligosaccharides with n=1-3 to the total weight of mannuronic acid oligosaccharides with n=4-7 is between 1.0-3.5.

C4. The use according to embodiment C1, wherein the total weight of mannuronic acid oligosaccharides with m+m'=1 or 2 is not less than 50%, preferably 60%-90%, more preferably 70%-90% of total weight of the composition.

C5. The use according to embodiment C4, wherein the total weight of mannuronic acid oligosaccharides with m+m'=1 is not less than 10%, preferably 30-40% of total weight of the composition.

C6. The use according to embodiment C4, wherein the total weight of mannuronic acid oligosaccharides with m+m'=2 is not less than 10%, preferably 30-50% of total weight of the composition.

C7. The use according to embodiment C1, wherein the total weight of mannuronic acid oligosaccharides with n=1-5 accounts for 80-95% of total weight of the composition.

C8. The use according to embodiment C1, wherein the total weight of mannuronic acid oligosaccharides with n=1-3 accounts for 20-70% of total weight of the composition.

C9. The use according to embodiment C3, wherein the ratio of the total weight of mannuronic acid oligosaccharides with n=1-3 to the total weight of mannuronic acid oligosaccharides with n=4-7 is between 1.0-3.0.

C10. The use according to any one of embodiments C1-C9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

C11. The use according to embodiment C10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

C12. The use according to embodiment C11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

C13. The use according to embodiment C1, wherein n is an integer selected from 1-19.

C14. The use according to embodiment C1, wherein n is an integer selected from 1-18.

C15. The use according to embodiment C1, wherein n is an integer selected from 1-17.

C16. The use according to embodiment C1, wherein n is an integer selected from 1-16.

C17. The use according to embodiment C1, wherein n is an integer selected from 1-15.

C18. The use according to embodiment C1, wherein n is an integer selected from 1-14.

C19. The use according to embodiment C1, wherein n is an integer selected from 1-13.

C20. The use according to embodiment C1, wherein n is an integer selected from 1-12.

C21. The use according to embodiment C1, wherein n is an integer selected from 1-11.

C22. The use according to embodiment C1, wherein n is an integer selected from 1-10.

C23. The use according to embodiment C1, wherein n is an integer selected from 1-9.

C24. The use of mannuronic acid oligosaccharide in the manufacture of a medicament for the treatment of Crohn's Disease, wherein the mannuronic acid oligosaccharide is a mannuronic acid oligosaccharide having formula (I) or a pharmaceutically acceptable salt thereof: 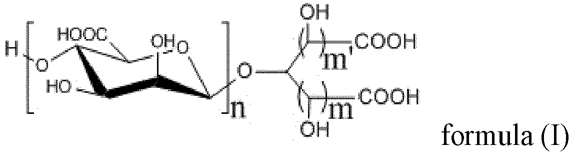 wherein n is an integer selected from 1-20, m is selected from 0, 1 or 2, and m' is selected from 0 or 1.

C25. The use according to embodiment C24, wherein n is an integer selected from 1-19.

C26. The use according to embodiment C24, wherein n is an integer selected from 1-18.

C27. The use according to embodiment C24, wherein n is an integer selected from 1-17.

C28. The use according to embodiment C24, wherein n is an integer selected from 1-16.

C29. The use according to embodiment C24, wherein n is an integer selected from 1-15.

C30. The use according to embodiment C24, wherein n is an integer selected from 1-14.

C31. The use according to embodiment C24, wherein n is an integer selected from 1-13.

C32. The use according to embodiment C24, wherein n is an integer selected from 1-12.

C33. The use according to embodiment C24, wherein n is an integer selected from 1-11.

C34. The use according to embodiment C24, wherein n is an integer selected from 1-10.

C35. The use according to embodiment C24, wherein n is an integer selected from 1-9.

C36. The use according to any one of embodiments C1-C35, wherein the pharmaceutically acceptable salt is a sodium salt or a potassium salt.

C37. A method for treating a patient having Crohn's Disease, the method comprises administering to the patient a therapeutically effective amount of the composition comprising mannuronic acid oligosaccharides defined according to any one of embodiments C1-C23 and C36 or the mannuronic acid oligosaccharide defined according to any one of embodiments C24-C36.

C38. The composition comprising mannuronic acid oligosaccharides defined according to any one of embodiments C1-C23 and C36 or the mannuronic acid oligosaccharides defined according to any one of embodiments C24-C36, for use in the treatment of Crohn's Disease.

### Embodiment D regarding Type 1 Diabetes

D1. The use of a composition comprising mannuronic acid oligosaccharides in the manufacture of a medicament for the treatment of Type 1 Diabetes, wherein the composition comprises a mannuronic acid oligosaccharide having formula (I) or a pharmaceutically acceptable salt thereof: 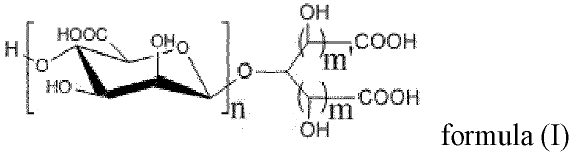
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

D2. The use according to embodiment D1, wherein the total weight of mannuronic acid oligosaccharides with n=1-2 accounts for 10-50%, preferably 25-50% of total weight of the composition.

D3. The use according to embodiment D1, wherein the ratio of the total weight of mannuronic acid oligosaccharides with n=1-3 to the total weight of mannuronic acid oligosaccharides with n=4-7 is between 1.0-3.5.

D4. The use according to embodiment D1, wherein the total weight of mannuronic acid oligosaccharides with m+m'=1 or 2 is not less than 50%, preferably 60%-90%, more preferably 70%-90% of total weight of the composition.

D5. The use according to embodiment D4, wherein the total weight of mannuronic acid oligosaccharides with m+m'=1 is not less than 10%, preferably 30-40% of total weight of the composition.

D6. The use according to embodiment D4, wherein the total weight of mannuronic acid oligosaccharides with m+m'=2 is not less than 10%, preferably 30-50% of total weight of the composition.

D7. The use according to embodiment D1, wherein the total weight of mannuronic acid oligosaccharides with n=1-5 accounts for 80-95% of total weight of the composition.

D8. The use according to embodiment D1, wherein the total weight of mannuronic acid oligosaccharides with n=1-3 accounts for 20-70% of total weight of the composition.

D9. The use according to embodiment D3, wherein the ratio of the total weight of mannuronic acid oligosaccharides with n=1-3 to the total weight of mannuronic acid oligosaccharides with n=4-7 is between 1.0-3.0.

D10. The use according to any one of embodiments D1-D9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

D11. The use according to embodiment D10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

D12. The use according to embodiment D11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

D13. The use according to embodiment D1, wherein n is an integer selected from 1-19.

D14. The use according to embodiment D1, wherein n is an integer selected from 1-18.

D15. The use according to embodiment D1, wherein n is an integer selected from 1-17.

D16. The use according to embodiment D1, wherein n is an integer selected from 1-16.

D17. The use according to embodiment D1, wherein n is an integer selected from 1-15.

D18. The use according to embodiment D1, wherein n is an integer selected from 1-14.

D19. The use according to embodiment D1, wherein n is an integer selected from 1-13.

D20. The use according to embodiment D1, wherein n is an integer selected from 1-12.

D21. The use according to embodiment D1, wherein n is an integer selected from 1-11.

D22. The use according to embodiment D1, wherein n is an integer selected from 1-10.

D23. The use according to embodiment D1, wherein n is an integer selected from 1-9.

D24. The use of mannuronic acid oligosaccharide in the manufacture of a medicament for the treatment of Type 1 Diabetes, wherein the mannuronic acid oligosaccharide is a mannuronic acid oligosaccharide having formula (I) or a pharmaceutically acceptable salt thereof: 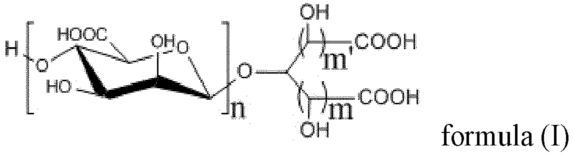 wherein n is an integer selected from 1-20, m is selected from 0, 1 or 2, and m' is selected from 0 or 1.

D25. The use according to embodiment D24, wherein n is an integer selected from 1-19.

D26. The use according to embodiment D24, wherein n is an integer selected from 1-18.

D27. The use according to embodiment D24, wherein n is an integer selected from 1-17.

D28. The use according to embodiment D24, wherein n is an integer selected from 1-16.

D29. The use according to embodiment D24, wherein n is an integer selected from 1-15.

D30. The use according to embodiment D24, wherein n is an integer selected from 1-14.

D31. The use according to embodiment D24, wherein n is an integer selected from 1-13.

D32. The use according to embodiment D24, wherein n is an integer selected from 1-12.

D33. The use according to embodiment D24, wherein n is an integer selected from 1-11.

D34. The use according to embodiment D24, wherein n is an integer selected from 1-10.

D35. The use according to embodiment D24, wherein n is an integer selected from 1-9.

D36. The use according to any one of embodiments D1-D35, wherein the pharmaceutically acceptable salt is a sodium salt or a potassium salt.

D37. A method for treating a patient having Type 1 Diabetes, the method comprises administering to the patient a therapeutically effective amount of the composition comprising mannuronic acid oligosaccharides defined according to any one of embodiments D1-D23 and D36 or the mannuronic acid oligosaccharide defined according to any one of embodiments D24-D36.

D38. The composition comprising mannuronic acid oligosaccharides defined according to any one of embodiments D1-D23 and D36 or the mannuronic acid oligosaccharides defined according to any one of embodiments D24-D36, for use in the treatment of Type 1 Diabetes.

### Embodiment E regarding Rheumatoid Arthritis

E1. The use of a composition comprising mannuronic acid oligosaccharides in the manufacture of a medicament for the treatment of Rheumatoid Arthritis, wherein the composition comprises a mannuronic acid oligosaccharide having formula (I) or a pharmaceutically acceptable salt thereof: 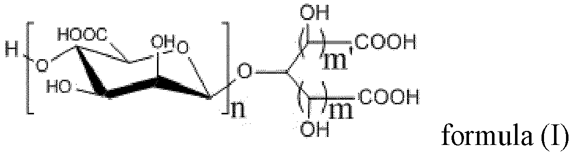
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

E2. The use according to embodiment E1, wherein the total weight of mannuronic acid oligosaccharides with n=1-2 accounts for 10-50%, preferably 25-50% of total weight of the composition.

E3. The use according to embodiment E1, wherein the ratio of the total weight of mannuronic acid oligosaccharides with n=1-3 to the total weight of mannuronic acid oligosaccharides with n=4-7 is between 1.0-3.5.

E4. The use according to embodiment E1, wherein the total weight of mannuronic acid oligosaccharides with m+m'=1 or 2 is not less than 50%, preferably 60%-90%, more preferably 70%-90% of total weight of the composition.

E5. The use according to embodiment E4, wherein the total weight of mannuronic acid oligosaccharides with m+m'=1 is not less than 10%, preferably 30-40% of total weight of the composition.

E6. The use according to embodiment E4, wherein the total weight of mannuronic acid oligosaccharides with m+m'=2 is not less than 10%, preferably 30-50% of total weight of the composition.

E7. The use according to embodiment E1, wherein the total weight of mannuronic acid oligosaccharides with n=1-5 accounts for 80-95% of total weight of the composition.

E8. The use according to embodiment E1, wherein the total weight of mannuronic acid oligosaccharides with n=1-3 accounts for 20-70% of total weight of the composition.

E9. The use according to embodiment E3, wherein the ratio of the total weight of mannuronic acid oligosaccharides with n=1-3 to the total weight of mannuronic acid oligosaccharides with n=4-7 is between 1.0-3.0.

E10. The use according to any one of embodiments E1-E9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

E11. The use according to embodiment E10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

E12. The use according to embodiment E11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

E13. The use according to embodiment E1, wherein n is an integer selected from 1-19.

E14. The use according to embodiment E1, wherein n is an integer selected from 1-18.

E15. The use according to embodiment E1, wherein n is an integer selected from 1-17.

E16. The use according to embodiment E1, wherein n is an integer selected from 1-16.

E17. The use according to embodiment E1, wherein n is an integer selected from 1-15.

E18. The use according to embodiment E1, wherein n is an integer selected from 1-14.

E19. The use according to embodiment E1, wherein n is an integer selected from 1-13.

E20. The use according to embodiment E1, wherein n is an integer selected from 1-12.

E21. The use according to embodiment E1, wherein n is an integer selected from 1-11.

E22. The use according to embodiment E1, wherein n is an integer selected from 1-10.

E23. The use according to embodiment E1, wherein n is an integer selected from 1-9.

E24. The use of mannuronic acid oligosaccharide in the manufacture of a medicament for the treatment of Rheumatoid Arthritis, wherein the mannuronic acid oligosaccharide is a mannuronic acid oligosaccharide having formula (I) or a pharmaceutically acceptable salt thereof: 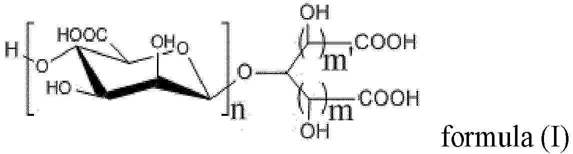 wherein n is an integer selected from 1-20, m is selected from 0, 1 or 2, and m' is selected from 0 or 1.

E25. The use according to embodiment E24, wherein n is an integer selected from 1-19.

E26. The use according to embodiment E24, wherein n is an integer selected from 1-18.

E27. The use according to embodiment E24, wherein n is an integer selected from 1-17.

E28. The use according to embodiment E24, wherein n is an integer selected from 1-16.

E29. The use according to embodiment E24, wherein n is an integer selected from 1-15.

E30. The use according to embodiment E24, wherein n is an integer selected from 1-14.

E31. The use according to embodiment E24, wherein n is an integer selected from 1-13.

E32. The use according to embodiment E24, wherein n is an integer selected from 1-12.

E33. The use according to embodiment E24, wherein n is an integer selected from 1-11.

E34. The use according to embodiment E24, wherein n is an integer selected from 1-10.

E35. The use according to embodiment E24, wherein n is an integer selected from 1-9.

E36. The use according to any one of embodiments E1-E35, wherein the pharmaceutically acceptable salt is a sodium salt or a potassium salt.

E37. A method for treating a patient having Rheumatoid Arthritis, the method comprises administering to the patient a therapeutically effective amount of the composition comprising mannuronic acid oligosaccharides defined according to any one of embodiments E1-E23 and E36 or the mannuronic acid oligosaccharide defined according to any one of embodiments E24-E36.

E38. The composition comprising mannuronic acid oligosaccharides defined according to any one of embodiments E1-E23 and E36 or the mannuronic acid oligosaccharides defined according to any one of embodiments E24-E36, for use in the treatment of Rheumatoid Arthritis.

### Embodiment F regarding Hashimoto's Thyroiditis

F1. The use of a composition comprising mannuronic acid oligosaccharides in the manufacture of a medicament for the treatment of Hashimoto's thyroiditis, wherein the composition comprises a mannuronic acid oligosaccharide having formula (I) or a pharmaceutically acceptable salt thereof: 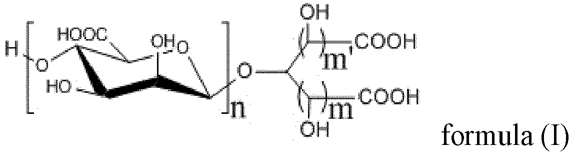
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

F2. The use according to embodiment F1, wherein the total weight of mannuronic acid oligosaccharides with n=1-2 accounts for 10-50%, preferably 25-50% of total weight of the composition.

F3. The use according to embodiment F1, wherein the ratio of the total weight of mannuronic acid oligosaccharides with n=1-3 to the total weight of mannuronic acid oligosaccharides with n=4-7 is between 1.0-3.5.

F4. The use according to embodiment F1, wherein the total weight of mannuronic acid oligosaccharides with m+m'=1 or 2 is not less than 50%, preferably 60%-90%, more preferably 70%-90% of total weight of the composition.

F5. The use according to embodiment F4, wherein the total weight of mannuronic acid oligosaccharides with m+m'=1 is not less than 10%, preferably 30-40% of total weight of the composition.

F6. The use according to embodiment F4, wherein the total weight of mannuronic acid oligosaccharides with m+m'=2 is not less than 10%, preferably 30-50% of total weight of the composition.

F7. The use according to embodiment F1, wherein the total weight of mannuronic acid oligosaccharides with n=1-5 accounts for 80-95% of total weight of the composition.

F8. The use according to embodiment F1, wherein the total weight of mannuronic acid oligosaccharides with n=1-3 accounts for 20-70% of total weight of the composition.

F9. The use according to embodiment F3, wherein the ratio of the total weight of mannuronic acid oligosaccharides with n=1-3 to the total weight of mannuronic acid oligosaccharides with n=4-7 is between 1.0-3.0.

F10. The use according to any one of embodiments F1-F9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

F11. The use according to embodiment F10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

F12. The use according to embodiment F11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

F13. The use according to embodiment F1, wherein n is an integer selected from 1-19.

F14. The use according to embodiment F1, wherein n is an integer selected from 1-18.

F15. The use according to embodiment F1, wherein n is an integer selected from 1-17.

F16. The use according to embodiment F1, wherein n is an integer selected from 1-16.

F17. The use according to embodiment F1, wherein n is an integer selected from 1-15.

F18. The use according to embodiment F1, wherein n is an integer selected from 1-14.

F19. The use according to embodiment F1, wherein n is an integer selected from 1-13.

F20. The use according to embodiment F1, wherein n is an integer selected from 1-12.

F21. The use according to embodiment F1, wherein n is an integer selected from 1-11.

F22. The use according to embodiment F1, wherein n is an integer selected from 1-10.

F23. The use according to embodiment F1, wherein n is an integer selected from 1-9.

F24. The use of mannuronic acid oligosaccharide in the manufacture of a medicament for the treatment of Hashimoto's thyroiditis, wherein the mannuronic acid oligosaccharide is a mannuronic acid oligosaccharide having formula (I) or a pharmaceutically acceptable salt thereof: 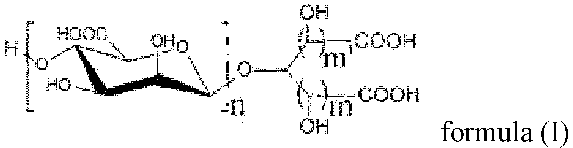 wherein n is an integer selected from 1-20, m is selected from 0, 1 or 2, and m' is selected from 0 or 1.

F25. The use according to embodiment F24, wherein n is an integer selected from 1-19.

F26. The use according to embodiment F24, wherein n is an integer selected from 1-18.

F27. The use according to embodiment F24, wherein n is an integer selected from 1-17.

F28. The use according to embodiment F24, wherein n is an integer selected from 1-16.

F29. The use according to embodiment F24, wherein n is an integer selected from 1-15.

F30. The use according to embodiment F24, wherein n is an integer selected from 1-14.

F31. The use according to embodiment F24, wherein n is an integer selected from 1-13.

F32. The use according to embodiment F24, wherein n is an integer selected from 1-12.

F33. The use according to embodiment F24, wherein n is an integer selected from 1-11.

F34. The use according to embodiment F24, wherein n is an integer selected from 1-10.

F35. The use according to embodiment F24, wherein n is an integer selected from 1-9.

F36. The use according to any one of embodiments F1-F35, wherein the pharmaceutically acceptable salt is a sodium salt or a potassium salt.

F37. A method for treating a patient having Hashimoto's thyroiditis, the method comprises administering to the patient a therapeutically effective amount of the composition comprising mannuronic acid oligosaccharides defined according to any one of embodiments F1-F23 and F36 or the mannuronic acid oligosaccharide defined according to any one of embodiments F24-F36.

F38. The composition comprising mannuronic acid oligosaccharides defined according to any one of embodiments F1-F23 and F36 or the mannuronic acid oligosaccharides defined according to any one of embodiments F24-F36, for use in the treatment of Hashimoto's thyroiditis.

### Embodiment G regarding Vitiligo

G1. The use of a composition comprising mannuronic acid oligosaccharides in the manufacture of a medicament for the treatment of Vitiligo, wherein the composition comprises a mannuronic acid oligosaccharide having formula (I) or a pharmaceutically acceptable salt thereof: 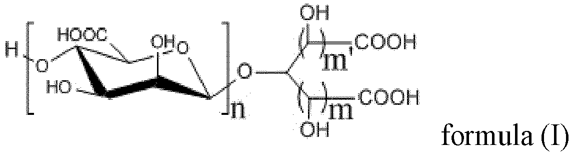
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

G2. The use according to embodiment G1, wherein the total weight of mannuronic acid oligosaccharides with n=1-2 accounts for 10-50%, preferably 25-50% of total weight of the composition.

G3. The use according to embodiment G1, wherein the ratio of the total weight of mannuronic acid oligosaccharides with n=1-3 to the total weight of mannuronic acid oligosaccharides with n=4-7 is between 1.0-3.5.

G4. The use according to embodiment G1, wherein the total weight of mannuronic acid oligosaccharides with m+m'=1 or 2 is not less than 50%, preferably 60%-90%, more preferably 70%-90% of total weight of the composition.

G5. The use according to embodiment G4, wherein the total weight of mannuronic acid oligosaccharides with m+m'=1 is not less than 10%, preferably 30-40% of total weight of the composition.

G6. The use according to embodiment G4, wherein the total weight of mannuronic acid oligosaccharides with m+m'=2 is not less than 10%, preferably 30-50% of total weight of the composition.

G7. The use according to embodiment G1, wherein the total weight of mannuronic acid oligosaccharides with n=1-5 accounts for 80-95% of total weight of the composition.

G8. The use according to embodiment G1, wherein the total weight of mannuronic acid oligosaccharides with n=1-3 accounts for 20-70% of total weight of the composition.

G9. The use according to embodiment G3, wherein the ratio of the total weight of mannuronic acid oligosaccharides with n=1-3 to the total weight of mannuronic acid oligosaccharides with n=4-7 is between 1.0-3.0.

G10. The use according to any one of embodiments G1-G9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

G11. The use according to embodiment G10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

G12. The use according to embodiment G11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

G13. The use according to embodiment G1, wherein n is an integer selected from 1-19.

G14. The use according to embodiment G1, wherein n is an integer selected from 1-18.

G15. The use according to embodiment G1, wherein n is an integer selected from 1-17.

G16. The use according to embodiment G1, wherein n is an integer selected from 1-16.

G17. The use according to embodiment G1, wherein n is an integer selected from 1-15.

G18. The use according to embodiment G1, wherein n is an integer selected from 1-14.

G19. The use according to embodiment G1, wherein n is an integer selected from 1-13.

G20. The use according to embodiment G1, wherein n is an integer selected from 1-12.

G21. The use according to embodiment G1, wherein n is an integer selected from 1-11.

G22. The use according to embodiment G1, wherein n is an integer selected from 1-10.

G23. The use according to embodiment G1, wherein n is an integer selected from 1-9.

G24. The use of mannuronic acid oligosaccharide in the manufacture of a medicament for the treatment of Vitiligo, wherein the mannuronic acid oligosaccharide is a mannuronic acid oligosaccharide having formula (I) or a pharmaceutically acceptable salt thereof: 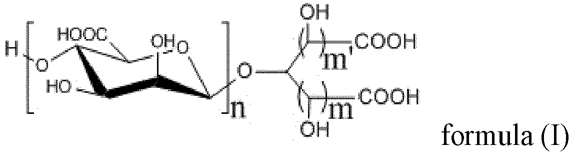 wherein n is an integer selected from 1-20, m is selected from 0, 1 or 2, and m' is selected from 0 or 1.

G25. The use according to embodiment G24, wherein n is an integer selected from 1-19.

G26. The use according to embodiment G24, wherein n is an integer selected from 1-18.

G27. The use according to embodiment G24, wherein n is an integer selected from 1-17.

G28. The use according to embodiment G24, wherein n is an integer selected from 1-16.

G29. The use according to embodiment G24, wherein n is an integer selected from 1-15.

G30. The use according to embodiment G24, wherein n is an integer selected from 1-14.

G31. The use according to embodiment G24, wherein n is an integer selected from 1-13.

G32. The use according to embodiment G24, wherein n is an integer selected from 1-12.

G33. The use according to embodiment G24, wherein n is an integer selected from 1-11.

G34. The use according to embodiment G24, wherein n is an integer selected from 1-10.

G35. The use according to embodiment G24, wherein n is an integer selected from 1-9.

G36. The use according to any one of embodiments G1-G35, wherein the pharmaceutically acceptable salt is a sodium salt or a potassium salt.

G37. A method for treating a patient having Vitiligo, the method comprises administering to the patient a therapeutically effective amount of the composition comprising mannuronic acid oligosaccharides defined according to any one of embodiments G1-G23 and G36 or the mannuronic acid oligosaccharide defined according to any one of embodiments G24-G36.

G38. The composition comprising mannuronic acid oligosaccharides defined according to any one of embodiments G1-G23 and G36 or the mannuronic acid oligosaccharides defined according to any one of embodiments G24-G36, for use in the treatment of Vitiligo.

### Embodiment H regarding Sjögren's Syndrome

H1. The use of a composition comprising mannuronic acid oligosaccharides in the manufacture of a medicament for the treatment of Sjögren's Syndrome, wherein the composition comprises a mannuronic acid oligosaccharide having formula (I) or a pharmaceutically acceptable salt thereof: 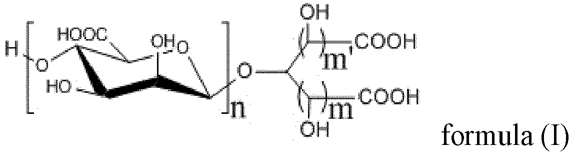
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

H2. The use according to embodiment HI, wherein the total weight of mannuronic acid oligosaccharides with n=1-2 accounts for 10-50%, preferably 25-50% of total weight of the composition.

H3. The use according to embodiment HI, wherein the ratio of the total weight of mannuronic acid oligosaccharides with n=1-3 to the total weight of mannuronic acid oligosaccharides with n=4-7 is between 1.0-3.5.

H4. The use according to embodiment HI, wherein the total weight of mannuronic acid oligosaccharides with m+m'=1 or 2 is not less than 50%, preferably 60%-90%, more preferably 70%-90% of total weight of the composition.

H5. The use according to embodiment H4, wherein the total weight of mannuronic acid oligosaccharides with m+m'=1 is not less than 10%, preferably 30-40% of total weight of the composition.

H6. The use according to embodiment H4, wherein the total weight of mannuronic acid oligosaccharides with m+m'=2 is not less than 10%, preferably 30-50% of total weight of the composition.

H7. The use according to embodiment HI, wherein the total weight of mannuronic acid oligosaccharides with n=1-5 accounts for 80-95% of total weight of the composition.

H8. The use according to embodiment HI, wherein the total weight of mannuronic acid oligosaccharides with n=1-3 accounts for 20-70% of total weight of the composition.

H9. The use according to embodiment H3, wherein the ratio of the total weight of mannuronic acid oligosaccharides with n=1-3 to the total weight of mannuronic acid oligosaccharides with n=4-7 is between 1.0-3.0.

H10. The use according to any one of embodiments H1-H9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

H11. The use according to embodiment H10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

H12. The use according to embodiment H11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

H13. The use according to embodiment HI, wherein n is an integer selected from 1-19.

H14. The use according to embodiment HI, wherein n is an integer selected from 1-18.

H15. The use according to embodiment HI, wherein n is an integer selected from 1-17.

H16. The use according to embodiment HI, wherein n is an integer selected from 1-16.

H17. The use according to embodiment HI, wherein n is an integer selected from 1-15.

H18. The use according to embodiment HI, wherein n is an integer selected from 1-14.

H19. The use according to embodiment HI, wherein n is an integer selected from 1-13.

H20. The use according to embodiment HI, wherein n is an integer selected from 1-12.

H21. The use according to embodiment HI, wherein n is an integer selected from 1-11.

H22. The use according to embodiment HI, wherein n is an integer selected from 1-10.

H23. The use according to embodiment HI, wherein n is an integer selected from 1-9.

H24. The use of mannuronic acid oligosaccharide in the manufacture of a medicament for the treatment of Sjögren's Syndrome, wherein the mannuronic acid oligosaccharide is a mannuronic acid oligosaccharide having formula (I) or a pharmaceutically acceptable salt thereof: 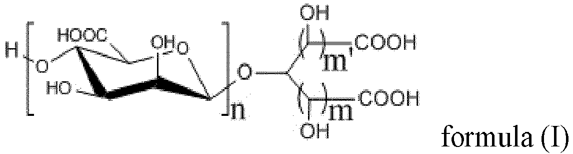 wherein n is an integer selected from 1-20, m is selected from 0, 1 or 2, and m' is selected from 0 or 1.

H25. The use according to embodiment H24, wherein n is an integer selected from 1-19.

H26. The use according to embodiment H24, wherein n is an integer selected from 1-18.

H27. The use according to embodiment H24, wherein n is an integer selected from 1-17.

H28. The use according to embodiment H24, wherein n is an integer selected from 1-16.

H29. The use according to embodiment H24, wherein n is an integer selected from 1-15.

H30. The use according to embodiment H24, wherein n is an integer selected from 1-14.

H31. The use according to embodiment H24, wherein n is an integer selected from 1-13.

H32. The use according to embodiment H24, wherein n is an integer selected from 1-12.

H33. The use according to embodiment H24, wherein n is an integer selected from 1-11.

H34. The use according to embodiment H24, wherein n is an integer selected from 1-10.

H35. The use according to embodiment H24, wherein n is an integer selected from 1-9.

H36. The use according to any one of embodiments H1-H35, wherein the pharmaceutically acceptable salt is a sodium salt or a potassium salt.

H37. A method for treating a patient having Sjögren's Syndrome, the method comprises administering to the patient a therapeutically effective amount of the composition comprising mannuronic acid oligosaccharides defined according to any one of embodiments H1-H23 and H36 or the mannuronic acid oligosaccharide defined according to any one of embodiments H24-H36.

H38. The composition comprising mannuronic acid oligosaccharides defined according to any one of embodiments H1-H23 and H36 or the mannuronic acid oligosaccharides defined according to any one of embodiments H24-H36, for use in the treatment of Sjögren's Syndrome.

### Embodiment I regarding polycystic ovary syndrome

11. The use of a composition comprising mannuronic acid oligosaccharides in the manufacture of a medicament for the treatment of Polycystic ovary syndrome, wherein the composition comprises a mannuronic acid oligosaccharide having formula (I) or a pharmaceutically acceptable salt thereof: 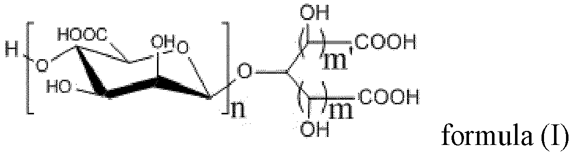
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

12. The use according to embodiment I1, wherein the total weight of mannuronic acid oligosaccharides with n=1-2 accounts for 10-50%, preferably 25-50% of total weight of the composition.

13. The use according to embodiment I1, wherein the ratio of the total weight of mannuronic acid oligosaccharides with n=1-3 to the total weight of mannuronic acid oligosaccharides with n=4-7 is between 1.0-3.5.

14. The use according to embodiment I1, wherein the total weight of mannuronic acid oligosaccharides with m+m'=1 or 2 is not less than 50%, preferably 60%-90%, more preferably 70%-90% of total weight of the composition.

15. The use according to embodiment 14, wherein the total weight of mannuronic acid oligosaccharides with m+m'=1 is not less than 10%, preferably 30-40% of total weight of the composition.

16. The use according to embodiment 14, wherein the total weight of mannuronic acid oligosaccharides with m+m'=2 is not less than 10%, preferably 30-50% of total weight of the composition.

17. The use according to embodiment I1, wherein the total weight of mannuronic acid oligosaccharides with n=1-5 accounts for 80-95% of total weight of the composition.

18. The use according to embodiment I1, wherein the total weight of mannuronic acid oligosaccharides with n=1-3 accounts for 20-70% of total weight of the composition.

19. The use according to embodiment 13, wherein the ratio of the total weight of mannuronic acid oligosaccharides with n=1-3 to the total weight of mannuronic acid oligosaccharides with n=4-7 is between 1.0-3.0.

110. The use according to any one of embodiments 11-19, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

111. The use according to embodiment 110, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

112. The use according to embodiment 111, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

113. The use according to embodiment I1, wherein n is an integer selected from 1-19.

114. The use according to embodiment I1, wherein n is an integer selected from 1-18.

115. The use according to embodiment I1, wherein n is an integer selected from 1-17.

116. The use according to embodiment I1, wherein n is an integer selected from 1-16.

117. The use according to embodiment I1, wherein n is an integer selected from 1-15.

118. The use according to embodiment I1, wherein n is an integer selected from 1-14.

119. The use according to embodiment I1, wherein n is an integer selected from 1-13.

120. The use according to embodiment I1, wherein n is an integer selected from 1-12.

121. The use according to embodiment I1, wherein n is an integer selected from 1-11.

122. The use according to embodiment I1, wherein n is an integer selected from 1-10.

123. The use according to embodiment I1, wherein n is an integer selected from 1-9.

124. The use of mannuronic acid oligosaccharide in the manufacture of a medicament for the treatment of Polycystic ovary syndrome, wherein the mannuronic acid oligosaccharide is a mannuronic acid oligosaccharide having formula (I) or a pharmaceutically acceptable salt thereof: 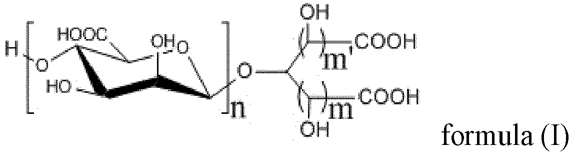 wherein n is an integer selected from 1-20, m is selected from 0, 1 or 2, and m' is selected from 0 or 1.

125. The use according to embodiment 124, wherein n is an integer selected from 1-19.

126. The use according to embodiment 124, wherein n is an integer selected from 1-18.

127. The use according to embodiment 124, wherein n is an integer selected from 1-17.

128. The use according to embodiment 124, wherein n is an integer selected from 1-16.

129. The use according to embodiment 124, wherein n is an integer selected from 1-15.

130. The use according to embodiment 124, wherein n is an integer selected from 1-14.

131. The use according to embodiment 124, wherein n is an integer selected from 1-13.

132. The use according to embodiment 124, wherein n is an integer selected from 1-12.

133. The use according to embodiment 124, wherein n is an integer selected from 1-11.

134. The use according to embodiment 124, wherein n is an integer selected from 1-10.

135. The use according to embodiment 124, wherein n is an integer selected from 1-9.

136. The use according to any one of embodiments 11-135, wherein the pharmaceutically acceptable salt is a sodium salt or a potassium salt.

137. A method for treating a patient having Polycystic ovary syndrome, the method comprises administering to the patient a therapeutically effective amount of the composition comprising mannuronic acid oligosaccharides defined according to any one of embodiments 11-123 and 136 or the mannuronic acid oligosaccharide defined according to any one of embodiments 124-136.

138. The composition comprising mannuronic acid oligosaccharides defined according to any one of embodiments 11-123 and 136 or the mannuronic acid oligosaccharides defined according to any one of embodiments 124-136, for use in the treatment of Polycystic ovary syndrome.

### Embodiment J regarding Celiac Disease

J1. The use of a composition comprising mannuronic acid oligosaccharides in the manufacture of a medicament for the treatment of Celiac Disease syndrome, wherein the composition comprises a mannuronic acid oligosaccharide having formula (I) or a pharmaceutically acceptable salt thereof: 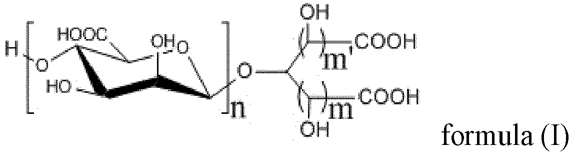
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

J2. The use according to embodiment J1, wherein the total weight of mannuronic acid oligosaccharides with n=1-2 accounts for 10-50%, preferably 25-50% of total weight of the composition.

J3. The use according to embodiment J1, wherein the ratio of the total weight of mannuronic acid oligosaccharides with n=1-3 to the total weight of mannuronic acid oligosaccharides with n=4-7 is between 1.0-3.5.

J4. The use according to embodiment J1, wherein the total weight of mannuronic acid oligosaccharides with m+m'=1 or 2 is not less than 50%, preferably 60%-90%, more preferably 70%-90% of total weight of the composition.

J5. The use according to embodiment J4, wherein the total weight of mannuronic acid oligosaccharides with m+m'=1 is not less than 10%, preferably 30-40% of total weight of the composition.

J6. The use according to embodiment J4, wherein the total weight of mannuronic acid oligosaccharides with m+m'=2 is not less than 10%, preferably 30-50% of total weight of the composition.

J7. The use according to embodiment J1, wherein the total weight of mannuronic acid oligosaccharides with n=1-5 accounts for 80-95% of total weight of the composition.

J8. The use according to embodiment J1, wherein the total weight of mannuronic acid oligosaccharides with n=1-3 accounts for 20-70% of total weight of the composition.

J9. The use according to embodiment J3, wherein the ratio of the total weight of mannuronic acid oligosaccharides with n=1-3 to the total weight of mannuronic acid oligosaccharides with n=4-7 is between 1.0-3.0.

J10. The use according to any one of embodiments J1-J9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

J11. The use according to embodiment J10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

J12. The use according to embodiment J11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

J13. The use according to embodiment J1, wherein n is an integer selected from 1-19.

J14. The use according to embodiment J1, wherein n is an integer selected from 1-18.

J15. The use according to embodiment J1, wherein n is an integer selected from 1-17.

J16. The use according to embodiment J1, wherein n is an integer selected from 1-16.

J17. The use according to embodiment J1, wherein n is an integer selected from 1-15.

J18. The use according to embodiment J1, wherein n is an integer selected from 1-14.

J19. The use according to embodiment J1, wherein n is an integer selected from 1-13.

J20. The use according to embodiment J1, wherein n is an integer selected from 1-12.

J21. The use according to embodiment J1, wherein n is an integer selected from 1-11.

J22. The use according to embodiment J1, wherein n is an integer selected from 1-10.

J23. The use according to embodiment J1, wherein n is an integer selected from 1-9.

J24. The use of mannuronic acid oligosaccharide in the manufacture of a medicament for the treatment of Celiac Disease syndrome, wherein the mannuronic acid oligosaccharide is a mannuronic acid oligosaccharide having formula (I) or a pharmaceutically acceptable salt thereof: 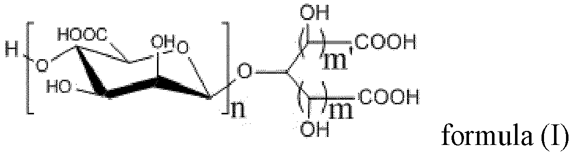 wherein n is an integer selected from 1-20, m is selected from 0, 1 or 2, and m' is selected from 0 or 1.

J25. The use according to embodiment J24, wherein n is an integer selected from 1-19.

J26. The use according to embodiment J24, wherein n is an integer selected from 1-18.

J27. The use according to embodiment J24, wherein n is an integer selected from 1-17.

J28. The use according to embodiment J24, wherein n is an integer selected from 1-16.

J29. The use according to embodiment J24, wherein n is an integer selected from 1-15.

J30. The use according to embodiment J24, wherein n is an integer selected from 1-14.

J31. The use according to embodiment J24, wherein n is an integer selected from 1-13.

J32. The use according to embodiment J24, wherein n is an integer selected from 1-12.

J33. The use according to embodiment J24, wherein n is an integer selected from 1-11.

J34. The use according to embodiment J24, wherein n is an integer selected from 1-10.

J35. The use according to embodiment J24, wherein n is an integer selected from 1-9.

J36. The use according to any one of embodiments J1-J35, wherein the pharmaceutically acceptable salt is a sodium salt or a potassium salt.

J37. A method for treating a patient having Celiac Disease syndrome, the method comprises administering to the patient a therapeutically effective amount of the composition comprising mannuronic acid oligosaccharides defined according to any one of embodiments J1-J23 and J36 or the mannuronic acid oligosaccharide defined according to any one of embodiments J24-J36.

J38. The composition comprising mannuronic acid oligosaccharides defined according to any one of embodiments J1-J23 and J36 or the mannuronic acid oligosaccharides defined according to any one of embodiments J24-J36, for use in the treatment of Celiac Disease syndrome.

### Embodiment K regarding Grave's disease

K1. The use of a composition comprising mannuronic acid oligosaccharides in the manufacture of a medicament for the treatment of Grave's disease, wherein the composition comprises a mannuronic acid oligosaccharide having formula (I) or a pharmaceutically acceptable salt thereof: 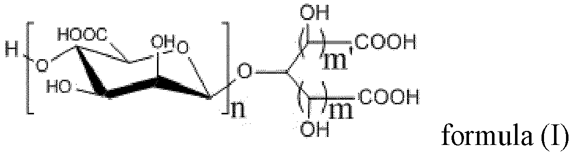
wherein n is an integer selected from 1-20, m is selected from 0, 1 or 2, m' is selected from 0 or 1,
and wherein,
the total weight of mannuronic acid oligosaccharides with n=1-5 accounts for more than 60% of the total weight of the composition;
the total weight of mannuronic acid oligosaccharides with n=1-2 accounts for less than 60% of the total weight of the composition.

K2. The use according to embodiment K1, wherein the total weight of mannuronic acid oligosaccharides with n=1-2 accounts for 10-50%, preferably 25-50% of total weight of the composition.

K3. The use according to embodiment K1, wherein the ratio of the total weight of mannuronic acid oligosaccharides with n=1-3 to the total weight of mannuronic acid oligosaccharides with n=4-7 is between 1.0-3.5.

K4. The use according to embodiment K1, wherein the total weight of mannuronic acid oligosaccharides with m+m'=1 or 2 is not less than 50%, preferably 60%-90%, more preferably 70%-90% of total weight of the composition.

K5. The use according to embodiment K4, wherein the total weight of mannuronic acid oligosaccharides with m+m'=1 is not less than 10%, preferably 30-40% of total weight of the composition.

K6. The use according to embodiment K4, wherein the total weight of mannuronic acid oligosaccharides with m+m'=2 is not less than 10%, preferably 30-50% of total weight of the composition.

K7. The use according to embodiment K1, wherein the total weight of mannuronic acid oligosaccharides with n=1-5 accounts for 80-95% of total weight of the composition.

K8. The use according to embodiment K1, wherein the total weight of mannuronic acid oligosaccharides with n=1-3 accounts for 20-70% of total weight of the composition.

K9. The use according to embodiment K3, wherein the ratio of the total weight of mannuronic acid oligosaccharides with n=1-3 to the total weight of mannuronic acid oligosaccharides with n=4-7 is between 1.0-3.0.

K10. The use according to any one of embodiments K1-K9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

K11. The use according to embodiment K10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

K12. The use according to embodiment K11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

K13. The use according to embodiment K1, wherein n is an integer selected from 1-19.

K14. The use according to embodiment K1, wherein n is an integer selected from 1-18.

K15. The use according to embodiment K1, wherein n is an integer selected from 1-17.

K16. The use according to embodiment K1, wherein n is an integer selected from 1-16.

K17. The use according to embodiment K1, wherein n is an integer selected from 1-15.

K18. The use according to embodiment K1, wherein n is an integer selected from 1-14.

K19. The use according to embodiment K1, wherein n is an integer selected from 1-13.

K20. The use according to embodiment K1, wherein n is an integer selected from 1-12.

K21. The use according to embodiment K1, wherein n is an integer selected from 1-11.

K22. The use according to embodiment K1, wherein n is an integer selected from 1-10.

K23. The use according to embodiment K1, wherein n is an integer selected from 1-9.

K24. The use of mannuronic acid oligosaccharide in the manufacture of a medicament for the treatment of Grave's disease, wherein the mannuronic acid oligosaccharide is a mannuronic acid oligosaccharide having formula (I) or a pharmaceutically acceptable salt thereof: 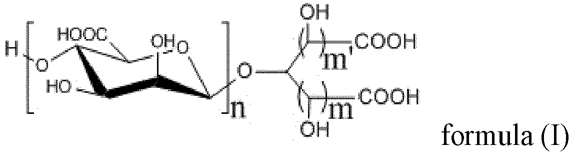 wherein n is an integer selected from 1-20, m is selected from 0, 1 or 2, and m' is selected from 0 or 1.

K25. The use according to embodiment K24, wherein n is an integer selected from 1-19.

K26. The use according to embodiment K24, wherein n is an integer selected from 1-18.

K27. The use according to embodiment K24, wherein n is an integer selected from 1-17.

K28. The use according to embodiment K24, wherein n is an integer selected from 1-16.

K29. The use according to embodiment K24, wherein n is an integer selected from 1-15.

K30. The use according to embodiment K24, wherein n is an integer selected from 1-14.

K31. The use according to embodiment K24, wherein n is an integer selected from 1-13.

K32. The use according to embodiment K24, wherein n is an integer selected from 1-12.

K33. The use according to embodiment K24, wherein n is an integer selected from 1-11.

K34. The use according to embodiment K24, wherein n is an integer selected from 1-10.

K35. The use according to embodiment K24, wherein n is an integer selected from 1-9.

K36. The use according to any one of embodiments K1-K35, wherein the pharmaceutically acceptable salt is a sodium salt or a potassium salt.

K37. A method for treating patients with Grave's disease, the method comprises administering to the patient a therapeutically effective amount of the composition comprising mannuronic acid oligosaccharides defined according to any one of embodiments K1-K23 and K36 or the mannuronic acid oligosaccharide defined according to any one of embodiments K24-K36.

K38. The composition comprising mannuronic acid oligosaccharides defined according to any one of embodiments K1-K23 and K36 or the mannuronic acid oligosaccharides defined according to any one of embodiments K24-K36, for use in the treatment of Grave's disease.

The present invention also relates to a composition comprising mannuronic acid oligosaccharides, which is a mixture of mannuronic acid oligosaccharides with different degrees of polymerization, and its main component is mannuronic acid oligosaccharides with a degree of polymerization of 2 to 10. It is known from the prior application that among the mannuronic acid oligosaccharides, the most active saccharides are penta- to deca- saccharides, especially hexasaccharides. However, different from the known prior art, the inventors found that adding a certain proportion of di- to tri- saccharides with lower activity into the most active penta- to decasaccharides would not reduce the biological activity or would even improve the biological activity under the same dosage having the same quality. Without being bound by any theory, it is speculated that this may be due to the fact that although the di- to tri- saccharides with smaller molecular weight cannot work alone, they can play a synergistic effect when mixed with other oligosaccharides. But when the proportion of di- to tri- saccharides is too high, the overall activity of the composition would still be reduced due to the low activity of di- to tri- saccharides *per se.* Therefore, the proportion of di- to tri-saccharides in the composition must be controlled within a certain range.

In the actual preparation process, a certain amount of di- to tri- saccharides would be produced in the oxidative degradation reaction. Because of its low activity, it is usually separated from the product and removed in order to avoid affecting the efficacy of the product. Based on the above findings of the inventors, it is unnecessary to separate and remove the di- to tri- saccharides in the oxidative degradation products, but is only needed to control the conditions of the oxidative degradation reaction, and control the proportion of di- to tri-saccharides within a certain range, and then the activity of the obtained composition is comparable to or even better than that of the composition disclosed in the prior application. In addition, because there is no need to remove di- to tri- saccharides as impurities, the product yield is theoretically significantly higher than the product yield disclosed in the prior application, the production costs are greatly reduced and the waste discharge is reduced. It is easier to be performed in actual production and easier to be performed in large-scale industrial production.

According to a preferred embodiment, the total weight of mannuronic acid oligosaccharides with m+m'=1 or 2 in the composition comprising mannuronic acid oligosaccharides of the present invention is not less than 50%, preferably 60% to 90%, more preferably 70%-90% of the total weight of the composition. In particular, in a preferred embodiment, the total weight of the mannuronic acid oligosaccharides with m+m'=1 in the composition comprising mannuronic acid oligosaccharides of the present invention is not less than 10%, preferably 30-40% of the total weight of the composition. In another preferred embodiment, the total weight of mannuronic acid oligosaccharides with m+m'=2 in the composition comprising mannuronic acid oligosaccharides of the present invention is not less than 10%, preferably 30-50% of the total weight of the composition.

According to a preferred embodiment, the total weight of mannuronic acid oligosaccharides with n=1-5 in the composition comprising mannuronic acid oligosaccharides of the present invention accounts for 80-95% of the total weight of the composition.

According to a preferred embodiment, the total weight of mannuronic acid oligosaccharides with n=1-2 in the composition comprising mannuronic acid oligosaccharides of the present invention accounts for 10-50%, preferably 25%-50% of the total weight of the composition.

According to a preferred embodiment, the total weight of mannuronic acid oligosaccharides with n=1-3 in the composition comprising mannuronic acid oligosaccharide of the present invention accounts for 20-70% of the total weight of the composition.

According to a preferred embodiment, the ratio of the total weight of mannuronic acid oligosaccharides with n=1-3 to the total weight of mannuronic acid oligosaccharides with n=4-7 in the composition comprising mannuronic acid oligosaccharide of the present invention is between 1.0-3.5, preferably 1.0-3.0.

According to a preferred embodiment, the weight percentages of mannuronic acid oligosaccharides with different degrees of polymerization in the composition comprising mannuronic acid oligosaccharides of the present invention are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%. In particular, the weight percentages of oligosaccharides in the composition are respectively: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%. More preferably, the weight percentages of oligosaccharides in the composition are respectively: disaccharides 10-20%, trisaccharides 18-30%, tetrasaccharides 15-28%, pentasaccharides 15-20%, hexasaccharides 5-10%, heptasaccharides 3-5%, octasaccharides 2-5%, nonasaccharides 1-3%, decasaccharides 1-3%.

In some embodiments, the pharmaceutically acceptable salts described herein are sodium or potassium salts.

The inventors found that when the oligosaccharides prepared above are compounded in a certain proportion, a highly active oligosaccharide composition can be obtained, and its activity is even higher than hexasaccharide, which is the most active; in particular, compositions with added disaccharides and trisaccharides in specific proportions are more active than compositions without disaccharides and trisaccharides. The proportion of each oligosaccharide in the highly active oligosaccharide composition needs to be combined according to the following proportional relationship:

The total weight of mannuronic acid oligosaccharides with n=1-5 in the composition accounts for more than 60%, preferably 80-95% of the total weight of the composition. The total weight of mannuronic acid oligosaccharides with n=1-2 accounts for less than 60%, preferably 10-50%, more preferably 25-50% of the total weight of the composition. The total weight of mannuronic acid oligosaccharides with n=1-3 accounts for 20-70% of the total weight of the composition. The ratio of the total weight of mannuronic acid oligosaccharides with n=1-3 to the total weight of mannuronic acid oligosaccharides with n=4-7 is between 1.0-3.5, more preferably 1.0-3.0.

The mannuronic acid oligosaccharide of the present invention can be formulated into drugs or pharmaceutical compositions together with one or more pharmaceutically acceptable carriers. The composition comprising mannuronic acid oligosaccharide according to the present invention may comprise one or more pharmaceutically acceptable carriers to be formulated into drugs or pharmaceutical compositions for use. The drugs or pharmaceutical compositions of the present invention can be in the form of tablets, hard capsules, soft capsules, enteric capsules, microcapsules, granules, syrups, injections, granules, emulsions, suspensions, solutions and sustained-release formulation for oral or non-oral administration.

The pharmaceutically acceptable carrier of the present invention refers to a pharmaceutically acceptable carrier known to those skilled in the art. The pharmaceutically acceptable carrier of the present invention includes, but is not limited to, fillers, wetting agents, binders, disintegrants, lubricants, adhesive, glidants, taste masking agents, surfactants, preservatives, etc. Fillers include, but are not limited to lactose, microcrystalline cellulose, starch, saccharide powder, dextrin, mannitol, calcium sulfate, etc. Wetting agents and binders include, but are not limited to sodium carboxymethylcellulose, hydroxypropyl cellulose, hydroxypropyl methylcellulose, gelatin, sucrose, polyvinylpyrrolidone, etc. Disintegrants include, but are not limited to sodium carboxymethyl starch, crosslinked polyvinylpyrrolidone, crosslinked sodium carboxymethyl cellulose, low substituted hydroxypropyl cellulose, etc. Lubricants include, but are not limited to, magnesium stearate, silica gel micropowder, talc, hydrogenated vegetable oil, polyethylene glycol, magnesium lauryl sulfate, etc. Adhesive includes, but are not limited to, arabic gum, alginic acid, calcium carboxymethylcellulose, sodium carboxymethylcellulose, glucose binders, dextrins, dextrose, ethyl cellulose, gelatin, liquid glucose, guar gum, hydroxyethylcellulose, hydroxypropyl cellulose, hydroxypropyl methylcellulose, magnesium aluminum silicate, maltodextrin, methylcellulose, polymethacrylates, polyvinylpyrrolidone, pregelatinized starch, sodium alginate, sorbitol, starch, syrup, and tragacanth gum. Glidants include, but are not limited to colloidal silica, powdered cellulose, magnesium trisilicate, silica and talc. Taste masking agents include, but are not limited to, aspartame, stevioside, fructose, glucose, syrup, honey, xylitol, mannitol, lactose, sorbitol, maltitol, and glycyrrhizin. Surfactants include, but are not limited to Tween -80 and poloxamer. Preservatives include, but are not limited to, parabens, sodium benzoate, potassium sorbate, etc.

As used herein, the term "treatment" generally refers to achieving a desired pharmacological and/or physiological effect. This effect can be preventive according to the complete or partial prevention of the disease or its symptoms; and/or can be therapeutic according to partial or complete stabilization or cure of the disease and/or side effects due to the disease. As used herein, "treatment" covers any treatment of a patient's disease, including: (a) prevention of diseases or symptoms occurring in patients who are susceptible to infection or symptoms but have not yet been diagnosed with the disease; (b) inhibiting the symptoms of the disease, i.e. preventing its development; or (c) relieving the symptoms of the disease, i.e. causing the disease or the deterioration of the symptoms. The term "therapeutically effective amount" as used herein refers to an amount sufficient to affect the treatment described herein.

The Th1-dominance related diseases described in the present invention can be selected from, but not limited to, Multiple Sclerosis, Crohn's Disease, Type 1 Diabetes, Rheumatoid Arthritis, Hashimoto's thyroiditis, Vitiligo, Sjögren's Syndrome, Polycystic ovarian syndrome (PCOS), Celiac Disease, Graves' disease.

The terms "Th1-dominance", "Th1-dominated", "Th1-cell-dominance" and "Th1-cell-dominated" are used interchangeably as used herein. In some cases, Th1-dominance can be represented by high peripheral blood Th1 cell levels. High peripheral blood Th1 cell levels may refer to peripheral blood Th1 cell levels in a subject that are higher than a suitable control (eg, a normal control, such as a normal human population) by 1%, 2%, 3%, 4%, 5%, 10%, 15%, 20%, 25%, 30%, 35%, 40%, 45%, 50%, 55%, 60%, 65%, 70%, 75%, 80%, 85%, 90%, 95%, 100%, 150%, 200%, 250%, 300%, 350%, 400%, 450%, 500%, 550%, 600%, 650%, 700%, 750%, 800%, 850%, 900%, 950%, 1000% or more, or the range constituted by any of the foregoing values as endpoints or any value therein, such as about 7% to about 28% and the like or about 7%, 14%, 21%, 28% and the like.

As used herein, the term "subject" refers to any living organism, including, but are not limited to, humans, non-human primates such as chimpanzees and other apes and monkeys, agricultural animals such as cattle, sheep, pigs, goats, and horses, domesticated mammals such as dogs and cats, laboratory animals, including rodents such as mice, rats, rabbits, guinea pigs and the like. The term does not designate a specific age and gender. In one embodiment, the subject is a human.

### Preparation of compositions comprising mannuronic acid oligosaccharides

In an exemplary embodiment, the preparation method of the composition comprising mannuronic acid oligosaccharide according to the present invention comprises the following steps:

### (1) Preparation of mannuronic acid oligosaccharide products:

Preparation of M fragement intermediates. As mentioned above, the raw material M segment intermediate used in the present invention can be prepared by methods known in the prior art. For example, Chinese patent applications CN98806637.8 ("Method for preparing uronic acid block from alginate") and CN02823707.2 ("Method for preparing alginate with high mannuronic acid content") disclose the methods. A common method can be simply described below: alginic acid is preliminarily degraded to give mixed polysaccharides of polymannuronic acid and polyguluronic acid; then the mixed polysaccharides are subjected to acidic precipitation to remove the polyguluronic acid therein, and further refinement is conducted to obtain a homopolymannuronic acid with a purity of more than 90%, i.e., an M-segment intermediate.

Ozone oxidative degradation. The M-segment intermediate is dissolved in an appropriate amount of water and stirred at room temperature or under heating condition. With continuous introduction of ozone, the reaction starts. The pH value of the reaction can be adjusted to 3-13, preferably 4-10, more preferably 6-8 by dropwise adding dilute hydrochloric acid or dilute NaOH solution. The temperature is preferably 0-70°Ce adjusted to 3-13, pref. After completion of the reaction, the introduction of ozone is stopped and the pH is adjusted to neutral.

Membrane separation and purification. The reaction product obtained above is formulated into a solution at a concentration of about 10% and separated by a molecular cut-off membrane to remove degradation products below monosaccharide. The retentate is collected. The MWCO of the molecular cut-off membrane used is 1000 Da - 3000 Da, preferably 2000 Da. The collected liquid is concentrated on a rotary evaporator and dried under vacuum to obtain an oligomannuronic acid oligosaccharide mixture. After analysis, it was found that these products are all compositions of di- to deca-oligosaccharide and their content is within a certain proportion range. Specific examples of the preparation method can be found in Examples 1-3, wherein three compositions A, B, and C were prepared according to the aforementioned methods.

### (2) Preparation of oligosaccharides with a single polymerization degree

The oligosaccharide mixture obtained in step (1) is dissolved to a concentration of about 10%, separated on a P6 gel chromatographic column, and subjected to ultraviolet detection to collect each effluent component. The components having the same polymerization degree are combined. Nine components of disaccharide to decasaccharide were collected, desalted by G10 gel column chromatography, concentrated by rotary evaporator, and dried in vacuum. A specific purification and preparation process is shown in Example 4. The operations such as column chromatography, desalting and drying are known to those skilled in the art.

### (3) Activity comparison of oligosaccharide composition

The pharmacological activity of the prepared composition was compared with the hexasaccharide purified in step (2) at the same time, and the results show that the oligosaccharide composition of the present invention is obviously better than the hexasaccharide with the best activity among the oligosaccharides with a single degree of polymerization, while compositions without disaccharides and trisaccharides were slightly less active than hexasaccharides. It can be seen that the combination of oligosaccharides with different degrees of polymerization can play a synergistic effect. When the proportion of di- to hexa- saccharide in the composition is more than 60%, and the proportion of di- and trisaccharide is not more than 60%, the activity of the composition is the highest; however, when the proportion of di- or tri-saccharides exceeds 60%, the activity of the composition will also decrease.

Advantages of the present invention are further illustrated in the following non-limiting examples. However, the specific materials and amounts thereof as well as other experimental conditions used in the examples should not be construed as limitation to the present invention. Unless otherwise specified, the parts, proportions, percentages, and the like in the present invention are all calculated by mass.

### Examples

### Example 1:

### Step 1): Preparation of mannuronic acid oligosaccharide mixture

An M-segment intermediate was prepared by the method disclosed in prior patents. The specific operations are briefly described below: 5 kg of sodium alginate was formulated into a solution of about 10%, and the pH was adjusted to about 3.0 by adding dilute hydrochloric acid. The solution was heated to 80 °C, and stirred. It was allowed to react for 10 hr before the heating was stopped. After cooling to room temperature, the pH was adjusted to 9.0 by adding NaOH, and further adjusted to 2.85 by adding dilute hydrochloric acid. The solution was centrifuged at 5000 rpm for 10 min. The supernatant was collected, and adjusted to pH 1.0 by adding HCl. After centrifugation, the precipitate was collected, concentrated on a rotary evaporator, and dried under vaccum to give 1500 g of the M-segment intermediate. 500 g of the M-segment intermediate was weighed, and dissolved in distilled water to prepare a solution in a volume of 5 L. The solution was adjusted to pH 6.5 with NaOH, and heated in a water bath to control the reaction temperature at 75 °C. The gas flow rate at the outlet of an oxygen cylinder and the power of an ozone generator were adjusted such that ozone was fed into the reaction solution at a mass concentration flow rate of 8 g/hr. After 4 hr of reaction, the feeding of ozone was stopped, and a suitable amount of water was added to adjust the concentration of the solution to about 10%. The solution was filtered through an ultrafiltration membrane with a molecular weight cut-off of 2,000 Da to collect a retentate. The collected retenate was concentrated on a rotary evaporator and dried under vacuum to obtain 350 g of mannuronic acid oligosaccharide product A.

### Step 2): Analysis of proportions and structures of oligosaccharides with various polymerization degrees in mannuronic acid oligosaccharide product A

100 mg of the above dried mannuronic acid oligosaccharide product A was accurately weighed, dissolved in water to a concentration of 10 mg/mL, and passed through a 0.22 µm filter membrane to obtain a test sample solution. The proportions of oligosaccharides with different polymerization degrees in the composition were determined by Superdex peptide molecular exclusion chromatography (GE Co.) in combination with multi-angle laser light scattering (MALS, Wyatt Co.). The experimental conditions were as follows:
Chromatographic column: Superdex peptide 10/300G1
Mobile phase: 0.1 mol/L NaCl
Injection volume: 10 µL
Flow rate: 0.3 mL/min
Test results: from disaccharide to decasaccharide were represented by dp2-dp10, respectively, dp2 was 19%, dp3 was 25%, dp4 was 22%, dp5 was 13%, dp6 was 9%, dp7 was 6%, dp8 was 3%, dp9 was 2% and dp10 was 1%.

### Step 3): LC-MS analysis of structures of oligosaccharides with various polymerization degrees in mannuronic acid oligosaccharide product A

Experimental conditions:
Chromatographic column: Superdex peptide 10/300G1
Mobile phase: 20% methanol + 80% 80 mmol/L NH4Ac
Flow rate: 0.1 mL/ min
Column temperature: 25°C ±lum°C ±
Mass spectrometry conditions: Agilent 6540 QTOF; ion source: ESI collision voltage 120 V; negative ion mode. The width of the acquired signal (m/z) was 100-1000.

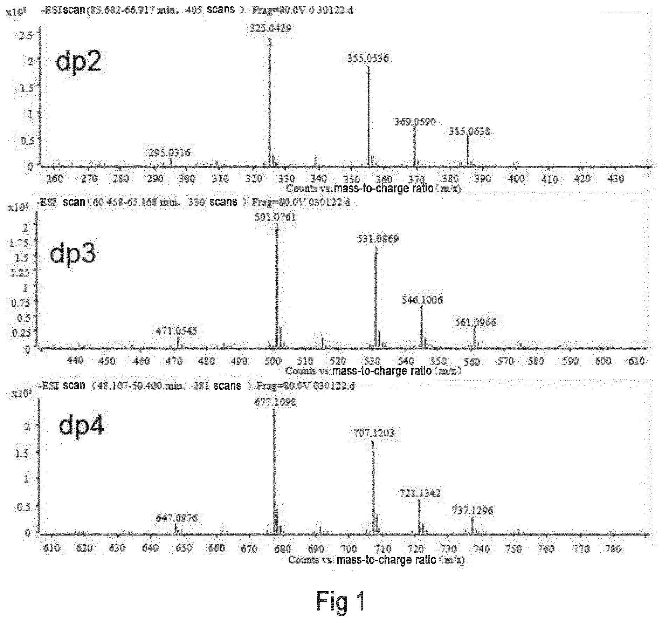

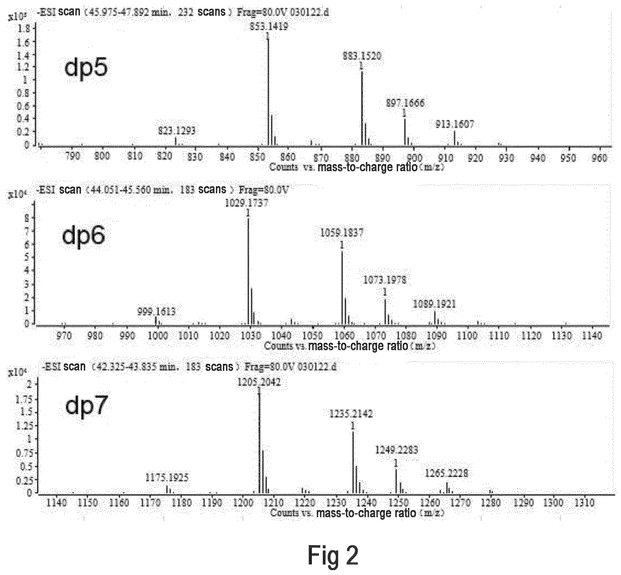

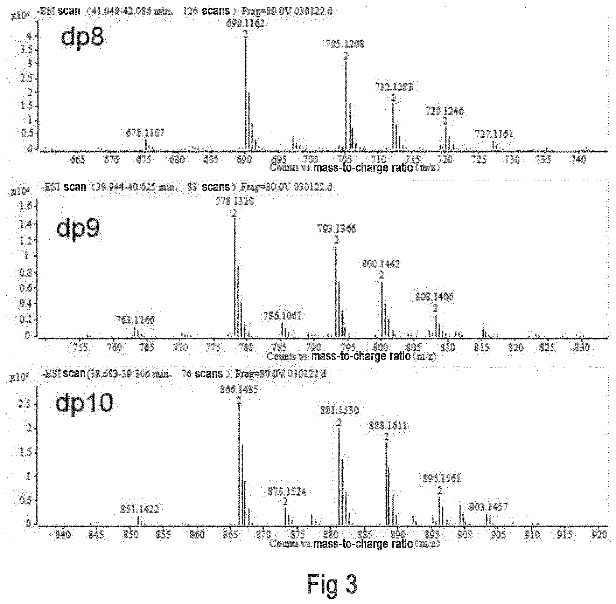

The mass spectra of oligosaccharides with various polymerization degrees are shown in Figures 1-3. Various signal peaks in the mass spectra were assigned, confirming the molecular structure of all oligosaccharides in product A, i.e., the structure shown in General Formula (I). See Table 1 below for the signal assignments and the structures corresponding to the signals.

**Table 1 Six structures of mannuronic acid oligosaccharides with different degrees of polymerization in product A and their mass-to-charge ratios in mass spectrometry**

| No. | Molecular Structure | molecular formula | mass-to-charge ratio (m/z) | | | | | | | | |
|---|---|---|---|---|---|---|---|---|---|---|---|
| | | | n=1 [M-1 ]⁻ | n=2 [M-1 ]⁻ | n=3 [M-1 ]⁻ | n=4 [M-1 ]⁻ | n=5 [M-1 ]⁻ | n=6 [M-1 ]⁻ | n=7 [M-2] ²⁻ | n=8 [M-2] ²⁻ | n=9 [M-2] ²⁻ |
| 1 | 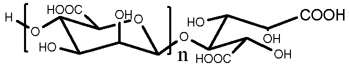 | (C₆H₈O₆)ₙ C₆H₁₀O₈ n=1-9 | 385 | 561 | 737 | 913 | 1089 | 1265 | 720 | 808 | 896 |
| 2 | 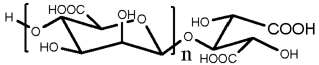 | (C₆H₈O₆)ₙ C₅H₈O₇ n=1-9 | 355 | 531 | 707 | 883 | 1059 | 1235 | 705 | 793 | 881 |
| 3 | 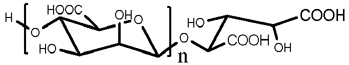 | (C₆H₈O₆)ₙ C₅H₈O₇ | 355 | 531 | 707 | 883 | 1059 | 1235 | 705 | 793 | 881 |
| | | n=1-9 | | | | | | | | | |
| 4 | 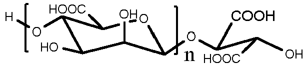 | (C₆H₈O₆)ₙ C₄H₆O₆ n=1-9 | 325 | 501 | 677 | 853 | 1029 | 1205 | 690 | 778 | 866 |
| 5 | 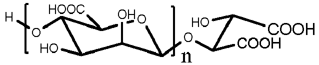 | (C₆H₈O₆)ₙ C₄H₆O₆ n=1-9 | 325 | 501 | 677 | 853 | 1029 | 1205 | 690 | 778 | 866 |
| 6 | 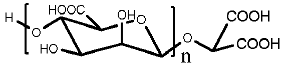 | (C₆H₈O₆)ₙ C₃H₄O₅ n=1-9 | 295 | 471 | 647 | 823 | 999 | 1175 | 675 | 763 | 851 |

It was found from the above mass spectrometric structural analysis that the mannuronic acid at the reducing end of the sugar chain in product A was oxidized to a saccharic acid structure (see General Formula I for the structure), which could be a mannaric acid structure comprising 6 carbon atoms (m+m'=3) with a content of about 10%~30%, or a decarboxylation product of mannaric acid, i.e., a saccharic dacid comprising 5 carbons (m+m'=2) (30~50%) and a saccharide diacid with 4 carbons (m+m'=1) (30%~40%).

### Example 2:

100 g of the M-segment intermediate in example 1 was weighed, and dissolved in distilled water to prepare a solution with a volume of 0.8 L. The solution was adjusted to pH 4.0 with NaOH, and the reaction was carried out at room temperature (25°C). The gas flow rate at the outlet of an oxygen cylinder and the power of an ozone generator were adjusted such that ozone was fed into the reaction solution at a mass concentration flow rate of 1 g/hr. After 10 hr of reaction, the feeding of ozone was stopped, and a suitable amount of water was added to adjust the concentration of the solution to about 15%. The solution was filtered through an ultrafiltration membrane with a molecular weight cut-off of 1,000 Da to collect a retentate. The collected retenate was concentrated on a rotary evaporator and dried under vacuum to obtain 80 g of mannuronic acid oligosaccharide product B.

The proportions of oligosaccharides components with various polymerization degrees in B were determined by Superdex peptide molecular exclusion chromatography (GE Co.) in combination with multi-angle laser light scattering (MALS, Wyatt Co.). The measurement method was the same as the relevant part in example 1. Test results: from disaccharide to decasaccharide were represented by dp2-dp10, respectively, dp2 was 20%, dp3 was 25%, dp4 was 19%, dp5 was 12%, dp6 was 9%, dp7 was 5%, dp8 was 5%, dp9 was 3% and dp10 was 2%.

### Example 3

100 g of the M-segment intermediate of example 1 was weighed, dissolved in distilled water to prepare a solution with a volume of 1.5 L. The solution was adjusted to pH 9.0 with NaOH, and the reaction was carried out in a water bath at 45 °C. The gas flow rate at the outlet of an oxygen cylinder and the power of an ozone generator were adjusted such that ozone was fed into the reaction solution at a mass concentration flow rate of 3 g/hr. After 2 hr of reaction, the feeding of ozone was stopped, and a suitable amount of water was added to adjust the concentration of the solution to about 5%. The solution was filtered through an ultrafiltration membrane with a molecular weight cut-off of 3,000 Da to collect a retentate. The collected retenate was concentrated on a rotary evaporator and dried under vacuum to obtain 60 g of mannuronic acid oligosaccharide product C.

The proportions of oligosaccharides with various polymerization degrees in C were determined by Superdex peptide molecular exclusion chromatography (GE Co.) in combination with multi-angle laser light scattering (MALS, Wyatt Co.). The measurement method was the same as the relevant part in example 1. Test results: from disaccharide to decasaccharide were represented by dp2-dp10, respectively, dp2 was 8%, dp3 was 20%, dp4 was 28%, dp5 was 19%, dp6 was 13%, dp7 was 6%, dp8 was 3%, dp9 was 2%, and dp10 was 1%.

### Example 4:

Preparation of mannuronic acid oligosaccharide with single polymerization degree, which was as follows:
1. Sample preparation: 300 g of mannuronic acid oligosaccharide product A prepared in example 1 was weighed, dissolved in water, prepared into 1000 mL of concentrated solution, and placed in a refrigerator at 4 p for use. For each use, 50 mL was taken out and was 1:2 diluted with water, and then suction filtered through a 0.22 µm ultrafiltration membrane.
2. Chromatographic separation conditions: The chromatograph was AKTA pure 150 (purchased from GE Co.) equipped with a UV detector and an automatic collector. Separation chromatographic column: 1.2 kg of BioGel P6 (purchased from Bio-Rad Co.) was mixed with deionized water, vacuum degassed, manually filled into a glass column (inner diameter: 10 cm), rinsed with 10 column volumes of pure water. The chromatographic column bed was stable and the height was 1.0 m. Then, the mobile phase was changed to a 0.02 M NaCl solution, and after equilibration with 10 column volumes, sample loading was initiated.
3. Sample loading and separation: The flow rate of the pump was set at 1 mL/min. After 100 mL of the sample solution was pumped to the top of the column through the chromatograph's own pump, it was switched to the mobile phase and eluted at a flow rate of 5 mL/min. After outflow of the dead water volume, automatic collection was initiated and 50 mL was collected per tube.
4. The sample loading was repeated, and after 20 repetitions of preparation, the same fractions were combined, concentrated on a rotary evaporator, and lyophilized to obtain a total of 9 oligosaccharides with single polymerization degree from disaccharide to decasaccharide.

### Example 5

A pharmacological activity evaluation was conducted between the compositions and hexasaccharide to examine the synergistic effect of the oligosaccharides with different polymerization degrees in the compositions and the range of proportions of the oligosaccharides.

### Sample preparation:

### Composition product D:

The mannuronic acid oligosaccharides with single polymerization degree as prepared in example 4 were accurately weighed from disaccharide to decasaccharide by the polymerization degree. The weight of each saccharide taken out was as follows: 3.0 g of disaccharide, 3.0 g of trisaccharide, 1.5 g of tetrasaccharide, 1.5 g of pentasaccharide, 0.4 g of hexasaccharide, 0.2 g of heptasaccharide, 0.2 g of octasaccharide, 0.1 g of nonasaccharide, and 0.1 g of decasaccharide. They were uniformly mixed to obtain 10 g of composition product D.

### Preparation of comparative experimental samples

A tetrasaccharide-to-decasaccharide containing mixture was prepared by referring to the methods disclosed in examples 1 and 2 of the prior patent application CN106344592A.

1g of sodium polymannuronate (weight average molecular weight 8235Da, provided by Shanghai Green Valley Pharmaceutical Co., Ltd.) was weighed and added with appropriate amount of distilled water to prepare 1% (weight percent) aqueous solution of sodium polymannuronate. The pH value of the 1% aqueous solution of sodium polymannuronate was adjusted to 4 with hydrochloric acid, and then the aqueous solution was placed in an autoclave. The reaction was subjected to heating at 110°C for 4 hours. The reacted solution was removed from the autoclave and allowed to cool. After cooling, the pH value of the reacted solution was adjusted with NaOH solution to obtain neutral liquid. Under the condition of stirring, the neutral liquid was slowly added into ethanol with a volume of 4 times the volume of the liquid. The alcohol precipitation was carried out, and the solution was left to stand overnight. The solid substance obtained by alcohol precipitation was filtered and separated, and the absolute ethanol was used to wash the solid substance obtained from filtering and separation during the filtering and separation process. Finally a white filter cake was produced. The filter cake was filtered in an oven at 60°C to obtain crude alginate oligosaccharide.

5g of crude alginate oligosaccharide was prepared into a 5% (weight percentage) aqueous solution. The fresh oxidant copper hydroxide was prepared by adding 25 ml of 5% (weight percent) copper sulfate solution to 50 ml of 10% (weight percent) sodium hydroxide solution and mixing immediately. The fresh oxidant copper hydroxide was immediately added to 40 ml of the above 5% (weight percent) alginate oligosaccharide solution, while heated in a boiling water bath until no more brick red precipitates were produced. The reaction system was centrifuged to remove the precipitate to obtain the supernatant. A little supernatant was added to the oxidant again to check whether there was still brick red precipitate produced. If brick red precipitate was still produced, all the supernatants obtained from the centrifugation would continue to react with other part of the oxidant until it was checked that no brick red precipitates were produced. The final reaction system was centrifuged to obtain the supernatant. 4 times the volume of 95% ethanol was added to the supernatant for alcohol precipitation, and the solution was allowed to stand overnight. The solid substance given by alcohol precipitation was filtered and separated, and the solid subtance was washed with absolute ethanol. The obtained solid substance was placed in an oven at 60°C and dried to give the crude alginate oligosaccharide represented by Formula (II).

1g of the crude alginate oligosaccharide was prepared into a 10% (weight percent) aqueous solution, and alcohol precipitation was carried out again by using a 95% ethanol solution. The precipitate obtained by alcohol precipitation again was filtered and separated, followed by optionally washing with absolute ethanol. The precipitate was separated and dried to obtain a solid substance. The solid substance was prepared into a 5% (weight percentage) aqueous solution. The aqueous solution was filtered with a 3 µ m pore size membrane and the filtrate was collected. The filtrate was eluted and separated on a molecular exclusion chromatography Bio-Gel-P6 gel column (1.6 x 180 cm, available from Bio-Rad Company). The eluent as mobile phase was 0.2 mol. L-1NH₄HCO₃. Eluate from the column chromatography was sequentially collected by a plurality of 5 ml test tubes, and then the saccharide content of the eluate in each test tube was detected by using a sulfuric acid-carbazole method. According to the detection results, eluates containing algin oligosaccharide components with different molecular weights were respectively collected. Eluates containing algin oligosaccharide components with different molecular weights were respectively concentrated under reduced pressure and lyophilized. Component 1 was discarded, and algin oligosaccharide components 2-12 were obtained, as shown in Formula (II) (n has a value of 0-10 respectively) with different molecular weights, and algin oligosaccharide eluate shown in Formula (II) with n=2-8 was collected, combined and dried. Algin oligosaccharide mixture (tetrasaccharide to decasaccharide mixture) shown in Formula (II) with n=2-8 was produced as a comparative experimental sample.

The proportion of oligosaccharide components with various polymerization degrees in comparative experimental samples was detected using Superdex peptide (GE Co.) molecular exclusion chromatography combined with multi-angle laser scattering (MALS, Wyatt) . The determination method is the same as the relevant part in example 1. Test results: tetrasaccharide to decasaccharide is represented by dp4-dp10, which is 10% dp4, 12% dp5, 13% dp6, 14% dp7, 15% dp8, 19% dp9 and 17% dp10, respectively.

Products A, B and C respectively prepared in examples 1, 2 and 3, product D in this example and oligosaccharide proportions in the comparative experimental samples are shown in Table 2 below.

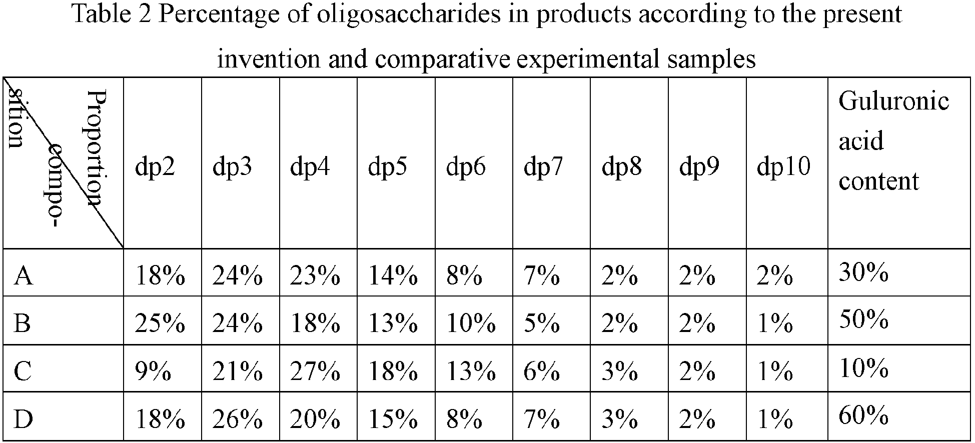

After testing, it was found that the mannuronic acid oligosaccharide and the composition comprising the mannuronic acid oligosaccharide of the present invention both acquired excellent therapeutic effects in the treatment of Th1-dominance related diseases.

After testing, it was found that the mannuronic acid oligosaccharide and the composition comprising the mannuronic acid oligosaccharide of the present invention both acquired excellent therapeutic effects in the treatment of Multiple Sclerosis diseases.

After testing, it was found that the mannuronic acid oligosaccharide and the composition comprising the mannuronic acid oligosaccharide of the present invention both acquired excellent therapeutic effects in the treatment of Crohn's Disease.

After testing, it was found that the mannuronic acid oligosaccharide and the composition comprising the mannuronic acid oligosaccharide of the present invention both acquired excellent therapeutic effects in the treatment of Type 1 Diabetes.

After testing, it was found that the mannuronic acid oligosaccharide and the composition comprising the mannuronic acid oligosaccharide of the present invention both acquired excellent therapeutic effects in the treatment of Rheumatoid Arthritis.

After testing, it was found that the mannuronic acid oligosaccharide and the composition comprising the mannuronic acid oligosaccharide of the present invention both acquired excellent therapeutic effects in the treatment of Hashimoto's thyroiditis.

After testing, it was found that the mannuronic acid oligosaccharide and the composition comprising the mannuronic acid oligosaccharide of the present invention both acquired excellent therapeutic effects in the treatment of Vitiligo.

After testing, it was found that the mannuronic acid oligosaccharide and the composition comprising the mannuronic acid oligosaccharide of the present invention both acquired excellent therapeutic effects in the treatment of Sjögren's Syndrome.

After testing, it was found that the mannuronic acid oligosaccharide and the composition comprising the mannuronic acid oligosaccharide of the present invention both acquired excellent therapeutic effects in the treatment of Polycystic ovarian syndrome.

After testing, it was found that the mannuronic acid oligosaccharide and the composition comprising the mannuronic acid oligosaccharide of the present invention both acquired excellent therapeutic effects in the treatment of Celiac Disease.

After testing, it was found that the mannuronic acid oligosaccharide and the composition comprising the mannuronic acid oligosaccharide of the present invention both acquired excellent therapeutic effects in the treatment of Graves's disease.

## Claims

1. The use of a composition comprising mannuronic acid oligosaccharides in the manufacture of a medicament for the treatment of Th1-dominance related diseases, wherein the composition comprises a mannuronic acid oligosaccharide having formula (I) or a pharmaceutically acceptable salt thereof: 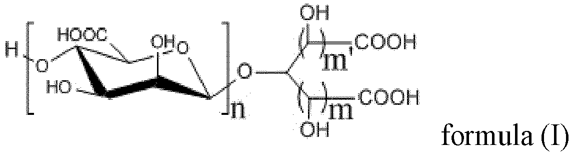
wherein n is an integer selected from 1-20, m is selected from 0, 1 or 2, m' is selected from 0 or 1,
and wherein,
the total weight of mannuronic acid oligosaccharides with n=1-5 accounts for more than 60% of total weight of the composition;
the total weight of mannuronic acid oligosaccharides with n=1-2 accounts for less than 60% of total weight of the composition.

2. The use according to claim 1, wherein the total weight of mannuronic acid oligosaccharides with n=1-2 accounts for 10-50%, preferably 25-50% of total weight of the composition.

3. The use according to claim 1, wherein the ratio of the total weight of mannuronic acid oligosaccharides with n=1-3 to the total weight of mannuronic acid oligosaccharides with n=4-7 is between 1.0-3.5.

4. The use according to claim 1, wherein the total weight of mannuronic acid oligosaccharides with m+m'=1 or 2 is not less than 50%, preferably 60%-90%, more preferably 70%-90% of total weight of the composition.

5. The use according to claim 4, wherein the total weight of mannuronic acid oligosaccharides with m+m'=1 is not less than 10%, preferably 30-40% of total weight of the composition.

6. The use according to claim 4, wherein the total weight of mannuronic acid oligosaccharides with m+m'=2 is not less than 10%, preferably 30-50% of total weight of the composition.

7. The use according to claim 1, wherein the total weight of mannuronic acid oligosaccharides with n=1-5 accounts for 80-95% of total weight of the composition.

8. The use according to claim 1, wherein the total weight of mannuronic acid oligosaccharides with n=1-3 accounts for 20-70% of total weight of the composition.

9. The use according to claim 3, wherein the ratio of the total weight of mannuronic acid oligosaccharides with n=1-3 to the total weight of mannuronic acid oligosaccharides with n=4-7 is between 1.0-3.0.

10. The use according to any one of claims 1-9, wherein the weight percentages of mannuronic acid oligosaccharides with different degrees of polymerization in the composition are: disaccharides 5-25%, trisaccharides 15-30%, tetrasaccharides 15-28%, pentasaccharides 5-25%, hexasaccharides 2-20%, heptasaccharides 2-20%, octasaccharides 2-20%, nonasaccharides 2-20%, decasaccharides 2-20%.

11. The use according to claim 10, wherein the weight percentages of mannuronic acid oligosaccharides with different degrees of polymerization in the composition are: disaccharides 5-25%, trisaccharides 15-30%, tetrasaccharides 15-28%, pentasaccharides 10-20%, hexasaccharides 5-15%, heptasaccharides 3-10%, octasaccharides 2-5%, nonasaccharides 1-5%, decasaccharides 1-5%.

12. The use according to claim 11, wherein the weight percentages of mannuronic acid oligosaccharides with different degrees of polymerization in the composition are: disaccharides 10-20%, trisaccharides 18-30%, tetrasaccharides 15-28%, pentasaccharides 10-20%, hexasaccharides 5-10%, heptasaccharides 3-5%, octasaccharides 2-5%, nonasaccharides 1-3%, decasaccharides 1-3%.

13. The use according to claim 1, wherein n is an integer selected from 1-19.

14. The use according to claim 1, wherein n is an integer selected from 1-18.

15. The use according to claim 1, wherein n is an integer selected from 1-17.

16. The use according to claim 1, wherein n is an integer selected from 1-16.

17. The use according to claim 1, wherein n is an integer selected from 1-15.

18. The use according to claim 1, wherein n is an integer selected from 1-14.

19. The use according to claim 1, wherein n is an integer selected from 1-13.

20. The use according to claim 1, wherein n is an integer selected from 1-12.

21. The use according to claim 1, wherein n is an integer selected from 1-11.

22. The use according to claim 1, wherein n is an integer selected from 1-10.

23. The use according to claim 1, wherein n is an integer selected from 1-9.

24. The use of a mannuronic acid oligosaccharide in the preparation of a medicament for the treatment of Th1-dominance related diseases, wherein the mannuronic acid oligosaccharide is a mannuronic acid oligosaccharide having formula (I) or a pharmaceutically acceptable salt thereof: 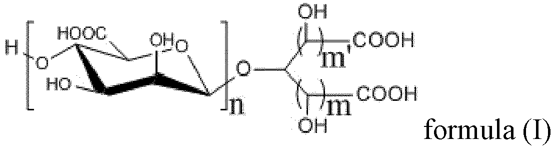 wherein n is an integer selected from 1-20, m is selected from 0, 1 or 2, and m' is selected from 0 or 1.

25. The use according to claim 24, wherein n is an integer selected from 1-19.

26. The use according to claim 24, wherein n is an integer selected from 1-18.

27. The use according to claim 24, wherein n is an integer selected from 1-17.

28. The use according to claim 24, wherein n is an integer selected from 1-16.

29. The use according to claim 24, wherein n is an integer selected from 1-15.

30. The use according to claim 24, wherein n is an integer selected from 1-14.

31. The use according to claim 24, wherein n is an integer selected from 1-13.

32. The use according to claim 24, wherein n is an integer selected from 1-12.

33. The use according to claim 24, wherein n is an integer selected from 1-11.

34. The use according to claim 24, wherein n is an integer selected from 1-10.

35. The use according to claim 24, wherein n is an integer selected from 1-9.

36. The use according to any one of claims 1-35, wherein the pharmaceutically acceptable salt is a sodium salt or a potassium salt.

37. A method for treating a patient having Th1-dominance related disease, comprising administering to the patient a therapeutically effective amount of the composition comprising mannuronic acid oligosaccharides defined according to any one of claims 1-23 and 36 or the mannuronic acid oligosaccharide defined according to any one of claims 24-36.

38. The composition comprising mannuronic acid oligosaccharides defined according to any one of claims 1-23 and 36 or the mannuronic acid oligosaccharides defined according to any one of claims 24-36, for use in the treatment of Th1-dominance related diseases.

39. The use according to any one of claims 1-36 or the method according to claim 37 or 38, wherein the Th1-dominance related disease is selected from Multiple Sclerosis, Crohn's Disease, Type 1 Diabetes, Rheumatoid Arthritis, Hashimoto's thyroiditis, Vitiligo, Sjögren's Syndrome, polycystic ovary syndrome, Celiac Disease, Grave's disease, preferably Multiple Sclerosis, Crohn's Disease and Rheumatoid Arthritis.
